# EUROPEAN PATENT APPLICATION

(11) **EP 4 660 299 A1**
(43) Date of publication of application: **10.12.2025**
(21) Application number: 24180379.0
(22) Date of filing: 06.06.2024
(51) Int. Cl.: C12N 5/0789, C12N 5/071

(54) **PRODUCTION OF BLOOD AND IMMUNE CELL PROGENITORS**

(71) Applicant: PHILIPPS-UNIVERSITÄT MARBURG, 35037 Marburg (DE)
(72) Inventor: Helker, Christian, 35043 Marburg (DE); Eberlein, Jean, 35043 Marburg (DE)
(74) Representative: Durm Patentanwälte PartG mbB

(57) **Abstract**

The invention relates to a method for producing progenitor cells of blood and immune cells, a cell population or a composition comprising blood and immune cells or progenitor cells thereof obtainable or obtained by the method of the invention, and the composition or cell population of the invention for use in transplantation.

## Description

The present invention relates to a method for producing progenitor cells of blood and immune cells, and to a cell population or composition comprising blood and immune cells or progenitor cells thereof, obtainable or obtained by the method of the invention. Further, the invention relates to the composition of the invention for use in transplantation and for use in treating a disease or disorder selected from the group consisting of hematologic disease, impaired hematopoiesis, pancytopenia, neutropenia, malignant disease, autoimmune disorder, immunodeficiency, malignancy, and metabolic disease.

### RELATED ART

The process of hematopoiesis is highly conserved among vertebrates and occurs in two waves (Galloway and Zon, 2003). The primitive wave gives rise to a transient population of erythroid and myeloid precursor cells (Herbomel, et al., 1999). During the definitive wave hemogenic endothelial cells (HECs) in the ventral wall of the dorsal aorta (VDA) differentiate into multipotent hematopoietic stem cells (HSC) (Bertrand et al., 2010a). These long-term multipotent hematopoietic stem and progenitor cells (HSPCs) can self-renew and differentiate to create all mature blood cells.

Hematopoietic stem cell transplantation (HSCT) including autologous hematopoietic stem cell transplantation (aHSCT) has evolved over the past years to treat a wide range of conditions, such as hematological cancers and autoimmune diseases. Owing to the potential of hematopoietic stem cells to generate the entire hematopoietic lineages, HSCT shows promising prospect for therapy of a broad-spectrum of hematological disorders. However, the limited number of HSCs in either bone marrow or umbilical cord blood limits their widespread use.

Ex vivo expansion of hematopoietic stem cells (HSCs) can help to overcome material shortage for transplantation purposes and genetic modification protocols. Approaches to expand HSCs in UCB have relied on cocktails of hematopoietic cytokines and factors, including UM171, the aryl hydrocarbon receptor antagonist SR1, deacetylase inhibitors, DNA methyltransferase inhibitors, Notch or Wnt ligands, PGE2, and others (Tajer et al., Cells. 2019, 8(2), 169). Different expansion protocols have been tested in clinical trials.

However, an optimal condition for ex vivo expansion of human HSCs still has not been found yet.

Hence, there is great interest in developing methods for in vitro or ex vivo production of blood and immune cells and progenitors thereof.

Apelin (Apln) is a small, secreted peptide, which was initially identified because of its inotropic activity (Szokodi et al., 2002) and subsequently described as a tip cell-enriched gene (del Toro et al., 2010). Apelin signaling via the Aplnr, a 7-transmembrane G-protein-coupled receptor (GPCR) is required for vascular development. The Apelin receptor is highly expressed in newly sprouting angiogenic blood vessel. Mouse and frog embryos lacking Apln or Aplnr function exhibit reduced vascular outgrowth, decreased EC proliferation, smaller vessel diameter as well as defects in the alignment of arteries and veins. Furthermore, Apelin signaling acts downstream of Notch signaling, where it is required for Notch-controlled angiogenesis (Helker et al. eLife 2020;9:e55589). However, the prior art is silent about the role of Apelin signaling in hematopoiesis.

### SUMMARY OF THE INVENTION

The invention refers to a method for production of progenitor cells of blood and immune cells, wherein Apelin signaling is inhibited. Particularly, the invention refers to a method for in vitro or ex vivo differentiation of endothelial cells or progenitors of endothelial cells, into progenitor cells of blood and immune cells, wherein Apelin signaling of endothelial cells is inhibited.

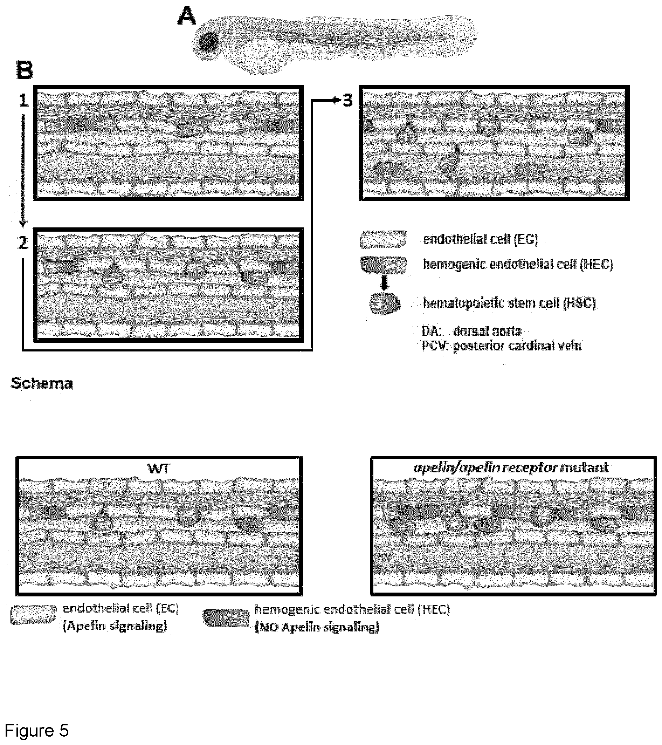

The inventors revealed an unknown role for Apelin signaling in restricting the formation of the hemogenic endothelium. In in vivo experiments, the inventors showed that modulation of the Apelin signaling pathway leads to increased differentiation and increased colonization of hematopoietic stem cells in the stem cell niche (Figure 5).

By live confocal imaging of novel transgenic reporters, the inventors observed the zonation of the DA into *Apelin receptor b (aplnrb)* expressing and *ap*/*nrb* non-expressing arterial endothelial cells (ECs). Of interest, aplnrb non-expressing ECs are exclusively located in the ventral floor of the dorsal aorta (VDA). Molecular analysis of HSC marker genes confirmed that these aplnrb-negative ECs give rise to HSCs. Genetic analysis revealed that the predefined hemogenic endothelium by the zonation of aplnrb expression is upstream of Gata2 and Runx1 function. Furthermore, zonation by aplnrb expression in the DA functionally restricts the hemogenic endothelium.

As shown by the inventors, loss of Apelin signaling leads to an expansion of the hemogenic endothelium (HE) on the expense of the vascular endothelium and subsequently to an increased formation of HSCs within the dorsal aorta (DA) (Figure 5). Endothelial cells within the DA lacking Apelin activity differentiate into HEs and subsequently HSCs. Conversely, forced activation of Apelin signaling in the vasculature leads to a decrease in HSC numbers in the DA. Thus, Apelin signaling functionally restricts the zonation of the hemogenic endothelium and regulates the number of HSCs emerging during embryonic development.

Hence, the data of the invention indicate that inhibition of the Apelin signaling pathway during the differentiation of hematopoietic stem cells increases the efficiency of this differentiation and results in a higher yield in the production of hematopoietic stem cells.

The present invention relates to, inter alia, the following items:
1. A method for producing progenitor cells of blood and immune cells, said method comprises
   (i) providing a starting population of cells, said starting population comprises endothelial cells and/or progenitor cells thereof, preferably said starting population comprises arterial endothelial cells and/or progenitor cells thereof; and
   (ii) differentiating said starting population into progenitor cells of blood and immune cells,
   wherein the endothelial cells and/or progenitor cells thereof are differentiated in presence of one or more agents capable of inhibiting or blocking Apelin signaling, capable of inhibiting or blocking expression of an Apelin receptor and/or capable of inhibiting or blocking expression of one or more down-stream activators of Apelin signaling.
2. The method of the preceding embodiment, wherein the endothelial or arterial endothelial cells are differentiated in presence of one or more agents capable of inhibiting or blocking Apelin signaling, capable of inhibiting or blocking expression of an Apelin receptor and/or capable of inhibiting or blocking expression of one or more down-stream activators of Apelin signaling.
3. The method of any one of the preceding embodiments, wherein progenitor cells of endothelial cells or arterial endothelial cells are selected from the group consisting of pluripotent stem cell, comprising embryonic stem cells and induced pluripotent stem cell (iPSC); mesodermal cells, preferably lateral mesodermal cells; primitive streak cells, preferably posterior primitive streak cells; hemangioblasts; and combinations thereof.
4. The method of any one of the preceding embodiments, wherein progenitor cells of endothelial cells or arterial endothelial cells are selected from the group consisting of pluripotent stem cells, excluding embryonic stem cells and including induced pluripotent stem cells (iPSC); mesodermal cells, preferably lateral mesodermal cells; primitive streak cells, preferably posterior primitive streak cells; hemangioblasts; and combinations thereof.
5. The method of any one of the preceding embodiments, wherein the cells of the starting population are HOXA+, preferably HOXA1+ to HOXA10+, more preferably HOXA1+, HOXA3+, and HOXA5+ to HOXA10+.
6. The method of any one of the preceding embodiments, wherein the progenitors of blood and immune cells are selected from the group consisting of hemogenic endothelial cells (HECs), hematopoietic progenitor cells (HPCs), hematopoietic stem cells (HSCs), and combinations thereof; preferably the progenitors of blood and immune cells are hemogenic endothelial cells (HECs) and/or hematopoietic progenitor cells (HPCs).
7. The method of any one of the preceding embodiments, wherein the progenitor cells of blood and immune cells express transcription factors HLF and/or HOXA, preferably HOXA is HOXA5, HOXA7, HOXA9, and HOXA10.
8. The method of any one of the preceding embodiments, wherein the agent capable of inhibiting or blocking Apelin signaling is an antagonist of an Apelin receptor or an antagonist of an Apelin receptor ligand.
9. The method of any one of the preceding embodiments, wherein the agent capable of inhibiting or blocking Apelin signaling is selected from the group consisting of an antibody, antibody fragment, small peptide, and small chemical compound.
10. The method of any one of the preceding embodiments, wherein the agent capable of inhibiting or blocking Apelin signaling is selected from the group consisting of protamine, protamine fragments and salts thereof; 4-oxo-6-((pyrimidin-2-ylthio)methyl)-4H-pyran-3-yl 4-nitrobenzoate (ML221), ALX 40-4C Trifluoroacetate, ALX 40-4C, ML339, (Ala13)-Apelin-13; Apelin-13 trifluoroacetate, MM54, MM107, MM108, MM193, MM262, MM297, MM298, MM299, MM300, MM301, MM302, MM312, MM313, MM314, MM315, MM316, MM412, MM413, MM414, MM415, MM416, MM417, MM418, MM419, MM420, MM421, MM422, MM423, MM424, MM426, MM428; an anti-APLNR ligand antibody, preferably JN241, JN126, JN126Fc, JN241Fc, JN126FcP13, and an anti-APLNR antagonist antibody, preferably H2aM9232N.
11. The method of any one of the preceding embodiments, wherein the agent capable of inhibiting or blocking expression of an Apelin receptor or expression of one or more down-stream activators of Apelin signaling is (i) a small interfering RNA molecule, or (ii) an antisense oligonucleotide.
12. The method of any one of the preceding embodiments, wherein the starting population is cultured in a medium comprising at least one growth factor, preferably the at least one growth factor is selected from the group consisting of bone morphogenetic proteins (BMPs), preferably BMP-4, Fibroblast Growth Factor 2 (FGF2), vascular endothelial growth factor (VEGF), insulin-like growth factor-1 (IGF-1), FMS-like tyrosine kinase ligand (FLT3-L), stem cell factor (SCF), IL-1, IL-3, IL-6, granulocyte colony-stimulating factor (G-CSF), thrombopoietin (TPO), granulocyte monocyte colony-stimulating factor (GM-SCF), macrophage colony-stimulating factor (M-CSF), megakaryocyte growth and development factor (MGDF), erythropoietin (EPO), IL-2, IL-4, IL-7, IL-11, and tumor necrosis factor alpha (TNFα), preferably the at least one growth factor is selected from the group consisting of bone morphogenetic proteins (BMPs), preferably 4 (BMP-4), Fibroblast Growth Factor 2 (FGF2), vascular endothelial growth factor (VEGF), and IL-6.
13. A cell population comprising cells progenitor cells of blood and immune cells, obtainable or obtained by the method of any one of the preceding embodiments, wherein preferably progenitor cells of blood and immune cells are hemogenic endothelial cells (HECs), hematopoietic progenitor cells (HPCs), hematopoietic stem cells (HSCs), and combinations thereof.
14. The cell population of the preceding embodiment, wherein the hemogenic endothelial cells are HOXA+, and the hematopoietic progenitor cells are HOXA+ and HLF+.
15. A composition comprising the cell population of the preceding embodiments and optionally a pharmaceutically acceptable carrier or cell medium.
16. The composition of any one of the preceding embodiments for use in transplantation of blood and immune cells of progenitor cells thereof in a mammalian subject, preferably a human subject.
17. The composition of the preceding embodiment for use in treating a disease or disorder selected from the group consisting of hematologic disease, impaired hematopoiesis, pancytopenia, neutropenia, autoimmune disorder, immunodeficiency, malignant disease, and metabolic disease; wherein preferably the pancytopenia or neutropenia is caused by chemotherapy, myeloablation, and/or radiation.
18. The composition for use according to any one of the preceding embodiments, wherein the patient is suffering from a disease selected from the group consisting of malignancies, preferably leukemias, such as acute myeloid leukemia, acute lymphoblastic leukemia, chronic myeloid leukemia, chronic lymphocytic leukemia; lymphomas, such as Hodgkin's lymphoma or non-Hodgkin's lymphoma; myelomas; metabolic diseases; hematopoietic disorders; hematologic diseases; metabolic disorders; environmentally induced diseases, preferably radiation poisoning or chemotherapy; myelodysplastic syndrome; viral diseases, preferably HTLV or HIV; autoimmune diseases; lysosomal storage disorders; immunodeficiencies, preferably inherited immunodeficiencies or acquired immunodeficiency syndromes; and hereditary skeletal dysplasia.
19. The composition for use according to any one of the preceding embodiments, wherein the patient is suffering from a hematopoietic disorder, wherein said hematopoietic disorder is preferably selected from the group consisting of acute myeloid leukemia, acute lymphoblastic leukemia, chronic myeloid leukemia, chronic lymphocytic leukemia, myeloproliferative disorders, myelodysplastic syndromes, multiple myeloma, non-Hodgkin lymphoma, Hodgkin disease, aplastic anemia, pure red cell aplasia, paroxysmal nocturnal hemoglobinuria, fanconi anemi, Thalassemia major, sickle cell anemia, severe combined immunodeficiency, Wiskott-Aldrich syndrome, and hemophagocytic lymphoh istiocytosis.
20. The composition for use according to any one of the preceding embodiments, wherein the patient is suffering from a hematologic disease, said hematologic disease is preferably selected from the group consisting of phagocyte disorders, such as myelodysplasia; anemias, preferably paroxysmal nocturnal hemoglobinuria (PNH; severe aplasia), aplastic anemia, acquired pure red cell aplasia; and myeloproliferative disorders, preferably polycythemia vera, essential thrombocytosis, myelofibrosis; hemoglobinopathies, sickle cell disease, β thalassemia major, cytopenias, preferably amegakaryocytic thrombocytopenia; and hemophagocytic syndromes, preferably hemophagocytic lymphoh istiocytosis.
21. The composition for use according to any one of the preceding embodiments, wherein the patient is suffering from an autoimmune disorder, said autoimmune disorders is preferably selected from the group consisting of multiple sclerosis, systemic lupus erythematosus and systemic sclerosis.
22. The composition for use according to any one of the preceding embodiments, wherein the patient is suffering from an immunodeficiency, said immunodeficiencies is preferably selected from the group consisting of T-cell deficiencies, such as ataxia-telangiectasia, DiGeorge syndrome; combined T- and B-cell deficiencies, such as severe combined immunodeficiency (SCID); Wiskott-Aldrich syndrome; phagocyte disorders, such as Kostmann syndrome, Shwachman-Diamond syndrome; immune dysregulation diseases, such as Griscelli syndrome, type II; and innate immune deficiencies, such as NF-Kappa-B Essential Modulator (NEMO) deficiency.
23. The composition for use according to any one of the preceding embodiments, wherein the patient is suffering from a metabolic disease, said metabolic disease is preferably selected from the group consisting of amyloidosis, preferably amyloid light chain amyloidosis; inborn errors of metabolism, lysosomal storage disorders, preferably Lipidoses including neuronal ceroid lipofuscinoses, infantile neuronal ceroid lipofuscinosis, Jansky-Bielschowsky disease (late infantile neuronal ceroid lipofuscinosis), sphingolipidoses, Niemann-Pick disease, Gaucher disease, leukodystrophies, adrenoleukodystrophy, metachromatic leukodystrophy, and Krabbe disease (globoid cell leukodystrophy), preferably the inborn errors of metabolism are selected from mucopolysaccharidosis, Gaucher disease, metachromatic leukodystrophies and adrenoleukodystrophies.
24. A biological material comprising blood or immune cells or progenitors thereof, such as hemogenic endothelial cells and/or hematopoietic progenitor cells , produced by the method of any one of the preceding embodiments.
25. A pharmaceutical composition comprising one or more agents capable of inhibiting or blocking Apelin signaling and a population of endothelial cells or progenitors of endothelial cells thereof, preferably the endothelial cells are arterial endothelial cells.
26. A kit for producing progenitor cells of blood and immune cells, comprising one or more agents capable of inhibiting or blocking Apelin signaling, capable of inhibiting or blocking expression of an Apelin receptor and/or capable of inhibiting or blocking expression of one or more down-stream activators of Apelin signaling; and instructions for use in a method of any one of the preceding embodiments and; optionally, a starting population of cells, said starting population comprises endothelial cells, progenitor cells thereof, growth factors, and/or cell culture media.

### DESCRIPTION OF FIGURES

The present invention is also illustrated by the following figures:

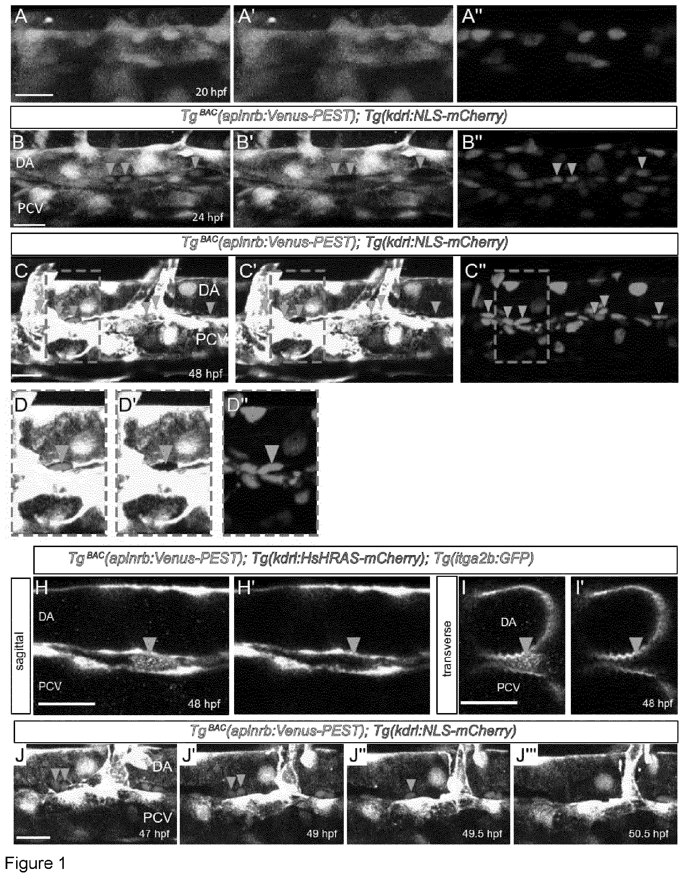

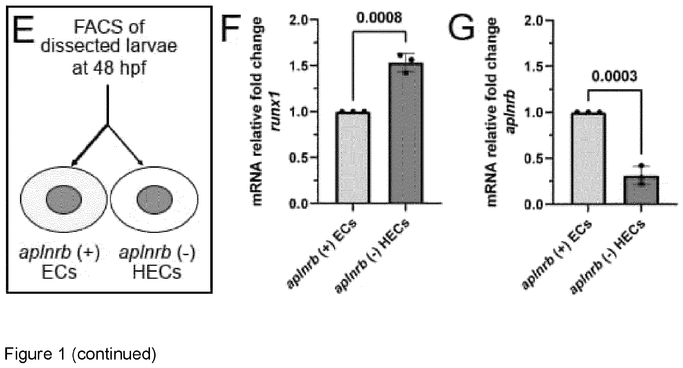

**Figure 1****:** aplnrb negative endothelial cells are hemogenic endothelial cells.
   (A-D) Confocal projection images of the trunk region of *Tg^{BAC}(aplnrb:Venus-PEST;* yellow*); Tg(kdrI:NLS-mCherry;* magenta) double transgenic zebrafish embryos at 20 hpf (A-A") and 24 hpf (B-B") and 48 hpf (C-D"). (B-D") *aplnrb:*Venus-PEST negative ECs can be detected in the ventral DA (white arrowheads) at 24 (B) and 48 hpf (C-D). (D-D") Magnification of the indicated region in C. (E) Schema displaying the FACS modalities for quantitative RT-PCR sample generation. (F, G) Quantitative RT-PCR results for *runx1* (unpaired t-test, p = 0.0008) and *aplnrb* (unpaired t-test, p = 0.0003) on FACS sorted *aplnrb*:Venus-PEST expressing and non-expressing ECs. (H-I') Sagittal and transverse slices of a confocal projection image of a triple transgenic *TgBAC(ap*/*nrb:Venus-PEST); Tg(kdrl:HsHRAS-mCherry); Tg(itga2b:GFP,* turquoise) zebrafish larva at 48 hpf. Sagittal (H) and transverse (I) slice showing a single ap/nrb:Venus-PEST negative HEC expressing *itga2b:GFP* and *kdrl*:HsHRAS-mCherry (white arrowhead). (J-J‴) Still images from confocal time-lapse movie of a double transgenic *Tg^{BAC}(aplnrb:Venus-PEST); Tg(kdrl:NLS-mCherry)* zebrafish larva. *aplnrb:*Venus-PEST negative ECs (arrowheads) bud out of the DA. DA, dorsal aorta; PCV, posterior cardinal vein; Scale bars: 20 µm.

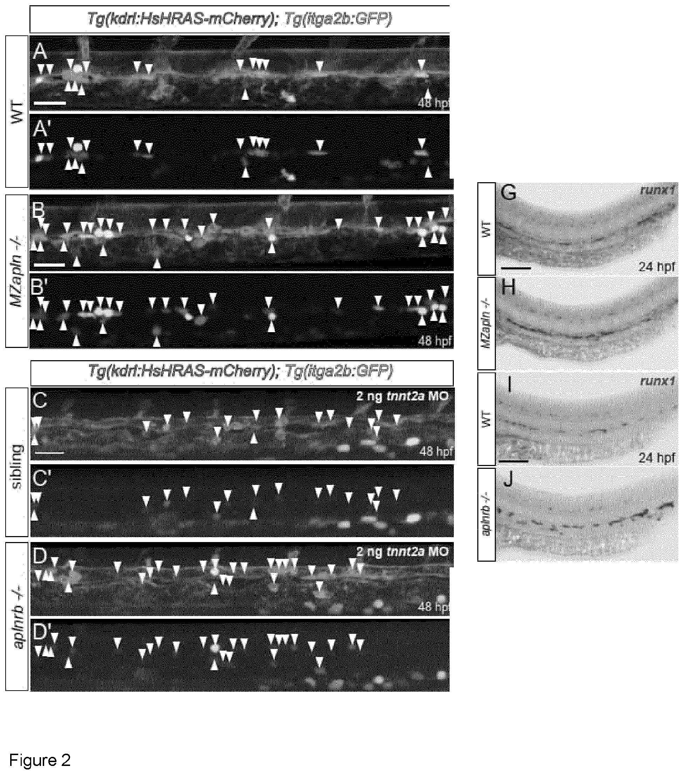

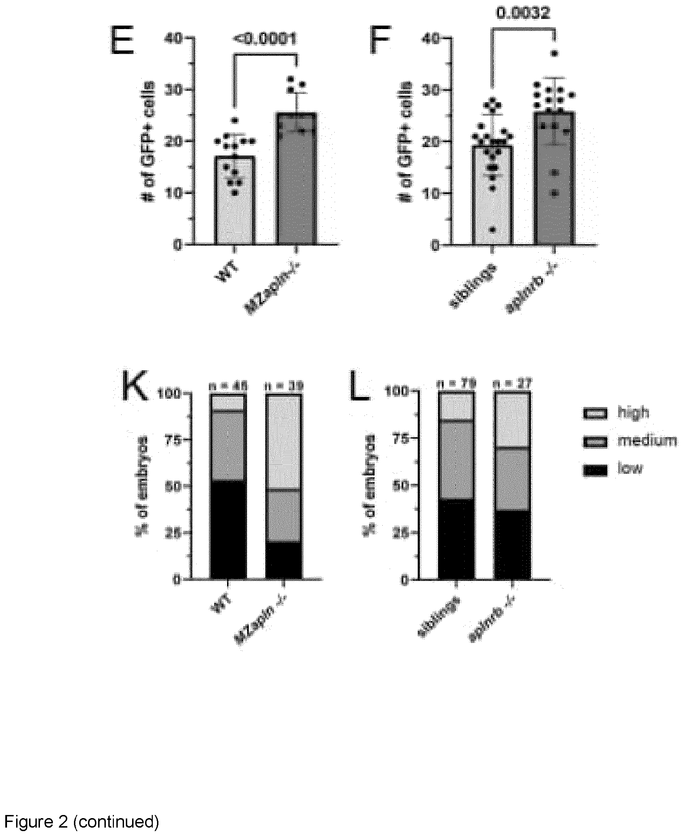

**Figure 2****:** Elevated HSC numbers in the VDA upon loss of Apelin signaling
   (A-D') Confocal projection images of double transgenic *Tg(kdrl:HsHRAS-mCherry); Tg(itga2b:GFP)* zebrafish larvae at 48 hpf. *itga2b*:GFP positive HSCs in the VDA are highlighted by white arrowheads. (A, A') Trunk region of a wildtype zebrafish larva. (B, B') Trunk region of a *MZapln* -/- mutant zebrafish larva. Elevated HSC numbers compared to WT larvae can be observed. (C, C') Trunk region of a wildtype zebrafish larva injected with 2 ng of a *tnnt2a* MO to stop bloodflow. (D, D') Trunk region of an *aplnrb* -/- mutant zebrafish larva injected with 2 ng of a *tnnt2a* MO. Elevated HSC numbers compared to WT larva can be observed. (E) Quantification of *itga2b:GFP* positive cells in the DA, corresponding to A and B (n = 24, unpaired t-test, p < 0.0001). (F) Quantification of *itga2b:GFP* positive cells in the DA, corresponding to C and D (n = 37, unpaired t-test, p = 0.0032). (G, H) WISH for *runx1* at 24 hpf in WT and *MZapln* -/- mutant zebrafish embryos, showing increased *runx1* expression in *MZapln* -/- mutant embryos. (I, J) WISH for *runx1* at 24 hpf in siblings and *aplnrb* -/- mutant zebrafish embryos, showing increased *runx1* expression in *aplnrb* -/- mutant embryos. (K) Phenotypic distribution plot of embryos in G and H (n = 84). (L) Phenotypic distribution plot of embryos in I and J (n = 106). HSC, hematopoietic stem cell; VDA, ventral dorsal aorta; DA, dorsal aorta; Scale bars: 30 µm (A-D); 100 µm (G-J).

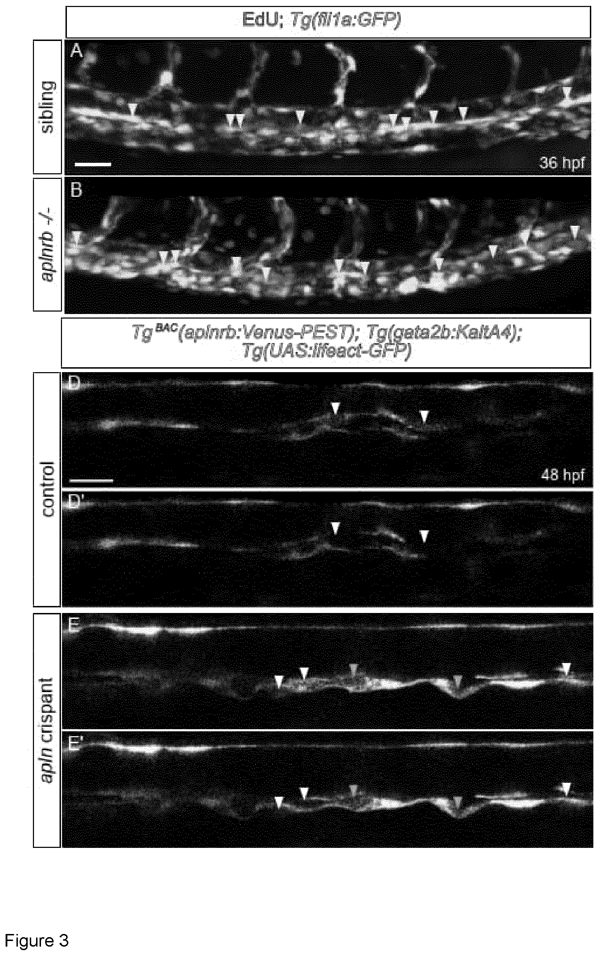

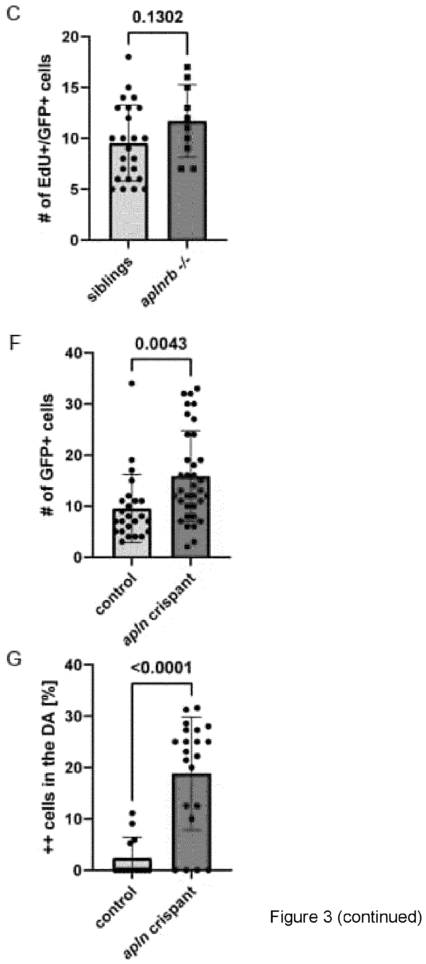

**Figure 3****:** Elevated HSC numbers in the DA upon loss of Apelin signaling are due to an extension of the HE
   (A-B) Confocal projection images of *Tg(fli1a:GFP)* zebrafish embryos at 36 hpf. Proliferating cells are labelled by EdU incorporation assay. White arrowheads label proliferating cells in the VDA. (A) Trunk region of a control sibling zebrafish embryo. (B) Trunk region of an *aplnrb* -/mutant zebrafish embryo. (C) Quantification of the proliferating cells in the VDA corresponding to A and B. (n=34, unpaired t-test, p = 0.1302). (D-E') Sagittal slices of confocal projection images of triple transgenic *Tg^{BAC}(ap*/*nrb:Venus-PEST);* Tg(gata2b:KaltA4); *Tg(UAS:lifeact-GFP*) zebrafish larvae at 48 hpf. (D,D') trunk region of a control zebrafish larva. *aplnrb* negative HECs/HSCs are highlighted by white arrowheads. (E,E') trunk region of a *apln* crispant injected zebrafish larva. *aplnrb* negative HECs/HSCs are highlighted by white arrowheads. *aplnrb* positive ectopic HECs/HSCs are highlighted by grey arrowheads. (F) Quantification of GFP+ HSCs in the DA, corresponding to D-E'. (n = 59, unpaired t-test, p = 0.0043). (G) Quantification of GFP/aplnrb:Venus-Pest double positive cells, displayed as percentage of total GFP+ cells. (n=34, unpaired t-test, p < 0.0001).

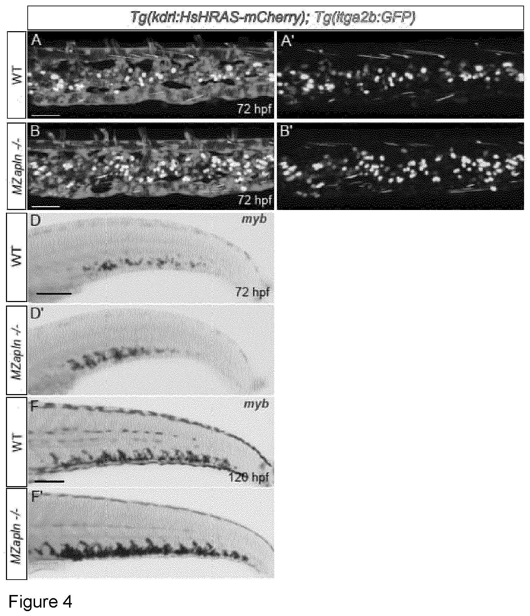

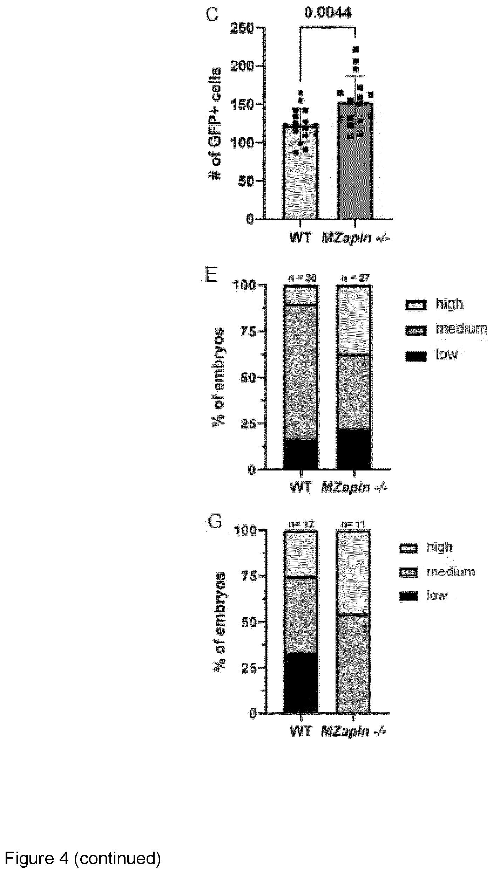

**Figure 4****:** Expanded hematopoietic stem cell pool can colonize the subsequent stem cell niche (A-B') Confocal projection images of double transgenic *Tg(kdrl:HsHRAS-mCherry); Tg(itga2b:GFP)* zebrafish larvae at 72 hpf. (D, D', F, F') WISH for *myb* on 72 and 120 hpf zebrafish larvae. (A, A') CVP region of a wildtype zebrafish larva. (B, B') CVP region of a *MZapln* -/- mutant zebrafish larva. Elevated HSC numbers compared to WT larvae can be observed. (C) Quantification of *itga2b:GFP* expressing cells in the CVP at 72 hpf, corresponding to A-B' (n = 32, unpaired t-test, p = 0.0044). (D) WISH for *myb* on 72 hpf WT larva. (D') WISH for *myb* on 72 hpf *MZapln* -/- mutant larva. Increased *myb* expression compared to WT can be observed in *MZapln* -/- mutant zebrafish larvae. (E) Phenotypic distribution plot of larvae in D/D' scored with low, medium and high *myb* expression in the CVP at 72 hpf (n = 57, Chi-square test, p=0.0249). (F) WISH for *myb* on 120 hpf WT larva. (F') WISH for *myb* on 120 hpf *MZapln* -/- mutant larva. Increased *myb* expression compared to WT can be observed in *MZapln* -/- mutant zebrafish larvae. (G) Phenotypic distribution plot of larvae in F/F' scored with low, medium and high *myb* expression in the CVP at 120 hpf (n = 42, Chi-square test, p=0.0017). CVP, caudal vein plexus; HSC, hematopoietic stem cell; Scale bars: 50 µm (A-B'); 100 µm (D, D', F, F').
**Figure 5****:** (A) Zebrafish; (B) Scheme of HSC differentiation; (C) Scheme of elevation of HSC numbers in the DA upon loss of Apelin signaling.
**Figure 6****:** Generation of hemogenic endothelial cells and hematopoietic progenitor cells from human iPSC within 10 days of differentiation.

### DETAILED DESCRIPTION OF THE INVENTION

In a first aspect, the invention relates to a method for producing progenitor cells of blood and immune cells, said method comprises
(i) providing a starting population of cells, said starting population comprises endothelial cells and/or progenitor cells thereof, preferably said starting population comprises arterial endothelial cells and/or progenitor cells thereof; and
(ii) culturing said starting population, wherein the endothelial cells and/or progenitor cells thereof are cultured in presence of one or more agents capable of inhibiting or blocking Apelin signaling, capable of inhibiting or blocking expression of an Apelin receptor, or capable of inhibiting or blocking expression of one or more down-stream activators of Apelin signaling.

One of the advantages of the production methods of the invention is that it enables the production of a high amount of progenitor cells of blood and immune cells in vitro from endothelial cells and/or progenitor cells thereof.

Culturing starting population of cells comprising endothelial cells and/or progenitor cells thereof in the presence of one or more agents capable of inhibiting or blocking Apelin signaling, capable of inhibiting or blocking expression of an Apelin receptor and/or capable of inhibiting or blocking expression of one or more down-stream activators of Apelin signaling, enables the production of hemogenic and hematopoietic cell types to provide a blood and immune cell populations, for example for short- and long-term engraftment in human patient in need thereof.

In certain embodiments the method for producing progenitor cells of blood and immune cells is an in vitro or ex vivo method.

### Starting population - Endothelial cells or progenitor cells thereof

The starting population comprises endothelial cells and/or progenitor cells thereof.

The starting population of cells can be derived from any source including adult, somatic, fetal or embryonic sources; genetically modified cells; cells from cell culture; immortalized hematopoietic stem cells; sources of pluripotent or multipotent cells, such as pluripotent stem cells; or any combination thereof.

The cells of the starting population can be autologous, allogeneic, xenogeneic, syngeneic or isogeneic cells. The cells of the starting population are preferably human cells.

In preferred embodiments, progenitor cells of endothelial cells and arterial endothelial cells are selected from the group consisting of embryonic stem cells, pluripotent stem cell, preferably induced pluripotent stem cell (iPSC), mesodermal cells, preferably lateral mesodermal cells, primitive streak cells, preferably posterior primitive streak cells and hemangioblasts and combinations thereof.

In preferred embodiments, the starting population does not comprise or does not consist of hemogenic endothelial cells, hematopoietic progenitors and/or hematopoietic stem cells.

In preferred embodiments, the cells of the starting population are HOXA+, preferably HOXA1-10+, more preferably HOXA1+, HOXA3+, and HOXA5+ to 10+.

Preferably, said endothelial cells and arterial endothelial cells are differentiated endothelial cells and differentiated arterial endothelial cells.

In preferred embodiments, the term "progenitors of endothelial cells" comprises pluripotent stem cells, including embryonic stem cells and induced pluripotent stem cell (iPSC); mesodermal cells, preferably lateral mesodermal cells; cells of the primitive streak, preferably the posterior primitive streak; and hemangioblasts as well as combinations thereof.

Preferably, the starting population of cells comprising endothelial cells or progenitors thereof are of human origin.

In preferred embodiments, the starting population of cells comprise or consist of ESCs or iPSCs, preferably IPSCs, more preferably human IPSCs. Preferably, ESCs or iPSCs are NANOG+, OCT4+, and/or SOX2+.

Examples of iPSCs comprise but are not limited to human skin primary fibroblasts, human fetal lung fibroblasts, human dermal fibroblasts and human peripheral blood mononuclear cells, human skin primary fibroblasts. Examples of iPSCs comprise but are not limited to the FD136-25, IMR90-16, Pci-1429 and Pci-CAU lines, LAM001-005 and LAM002-002 lines.

Preferably, the starting population of cells comprising endothelial cells or progenitors thereof are obtained by methods without destroying embryos. Technology to obtain the starting population while preserving the viability of the donor embryo are known in the prior art (for example, see Klimanskaya et al., 2006, Nature, 444:481-485 and Y. Chung, et al., Cell Stem Cell, 2, 113-117, 2008; Dittrich et al., Geburtshilfe Frauenheilkd. 2015 Dec; 75(12): 1239-1242, etc.). Preferably, any method, product or use that requires destruction of a human embryo is disclaimed.

Preferably, the starting population of cells comprises pluripotent stem cells. Pluripotent stem cells are preferably defined herein as cells that have the capacity to self-renew by dividing and to develop into the three primary germ cell layers of the early embryo and therefore into all cells of the adult body, but not extra-embryonic tissues such as the placenta.

Preferably, the starting population of cells comprises embryonic stem cells. Embryonic stem cells (ESCs) are preferably defined herein as a type of pluripotent stem cells (see above) derived from the inner cell mass of a blastocyst, the early mammalian embryo that implants into the uterus. The term includes patient-matched embryonic stem cell lines that are derived using somatic cell nuclear transfer. Embryonic stem cells can self-renew, infinitely proliferate, and broadly differentiate into all cells of the adult body, including mesodermal cells and their descendants.

Preferably, the starting population of cells comprises induced pluripotent stem cells. Induced pluripotent stem cells (iPSCs) are preferably defined herein as a type of pluripotent stem cell derived from a non-pluripotent cell, typically a somatic cell, by inducing expression of certain genes and transcription factors that can convert somatic cells into pluripotent stem cells (e.g., doi:10.1016/j.cell.2007.11.019; doi:10.1126/science.1151526). iPSCs exhibit similar traits to those of embryonic stem cells (ESCs) but do not require the use of embryos. Since iPSCs can be derived directly from adult tissues, they not only bypass the need for embryos, but can be made in a patient-matched manner, which means that each individual could have their own pluripotent stem cell line. These unlimited supplies of autologous cells could be used to generate transplants without the risk of immune rejection. Thus, in certain embodiments, the starting population of cells comprises pluripotent stem cells derived from somatic cells, e.g., by generated by somatic cell reprogramming.

Preferably, the starting population of cells comprises cells of the primitive streak, preferably the posterior primitive streak. Cells of the primitive streak are preferably BRACHYURY+ and/or MIXL1+. In another preferred embodiment, cells of the posterior primitive streak are HOXA+. In another preferred embodiment, cells of the posterior primitive streak are BRACHYURY+, MIXL1+, CDX2+, CDX4+ and/or HOXA+.

Preferably, the starting population of cells comprises mesodermal cells, preferably lateral mesodermal cells. Mesodermal and lateral mesodermal cells are preferably ETV2+, SCL/TAL1 +, LMO2+ and/or FLI1+. Mesodermal and lateral mesodermal cells are preferably HOXA+. Mesodermal and lateral mesodermal cells are preferably ETV2+, SCL/TAL1+, LMO2+, FLI1 + and/or HOXA+.

Mesodermal, lateral mesodermal, primitive streak and posterior primitive streak cells can be differentiated in vitro from induced pluripotent stem cells and embryonic stem cells (doi: 10.1016/j.devcel.2024.03.003 and doi: 10.1007/s12185-010-0518-8). Mesodermal cells and primitive streak cells give rise to the endothelium of blood vessels, red and white blood cells and other cell and tissue types.

In preferred embodiments, the endothelial cells and arterial endothelial cells are CD34+, CD144+, SOX17+, and/or DLL4+. In preferred embodiments, the endothelial cells and arterial endothelial cells are CD34+, CD144+, SOX17+, DLL4+; and/or HOXA+. In preferred embodiments, the endothelial cells and arterial endothelial cells are HOXA+, preferably HOXA1-10+, more preferably HOXA1+, HOXA3+, and HOXA5-10+ or HOXA5+, HOXA7+, HOXA9+, and HOXA10+.

In preferred embodiments, the cells of the starting population are HOXA+, i.e. HOXA genes were expressed, preferably HOXA1-10, more preferably HOXA1, HOXA3, and HOXA5-10. In more preferred embodiments, the cells of the starting population are HOXA5+, HOXA7+, HOXA9+, and HOXA10+.

In preferred embodiments, the starting population of cells comprise or consist of ESCs or iPSCs, preferably IPSCs, more preferably human IPSCs;
wherein ESCs or IPSCs are differentiated into primitive streak cells, preferably posterior primitive streak cells;
the primitive or posterior primitive streak cells are differentiated into mesodermal cells, preferably lateral mesodermal cells;
the mesodermal or lateral mesodermal cells are differentiated into endothelial cells, preferably arterial endothelial cells;
the endothelial or arterial endothelial cells are differentiated into hemogenic endothelial cells,
wherein the endothelial cells and/or progenitor cells thereof, preferably the endothelial cells, more preferably the arterial endothelial cells are differentiated in presence of one or more agents capable of inhibiting or blocking Apelin signaling, capable of inhibiting or blocking expression of an Apelin receptor, and/or capable of inhibiting or blocking expression of one or more down-stream activators of Apelin signaling;
optionally the hemogenic endothelial cells are differentiated into hematopoietic progenitor cells.

In other preferred embodiments, the starting population of cells are primitive streak cells, preferably posterior primitive streak cells; wherein the primitive streak cells are differentiated into mesodermal cells, preferably lateral mesodermal cells; the mesodermal or lateral cells are differentiated into endothelial cells, preferably arterial endothelial cells; the endothelial or arterial endothelial cells are differentiated into hemogenic endothelial cells, wherein one or more agents capable of inhibiting or blocking Apelin signaling, capable of inhibiting or blocking expression of an Apelin receptor and/or capable of inhibiting or blocking expression of one or more down-stream activator of Apelin signaling is added to the endothelial cells; optionally the hemogenic endothelial cells are differentiated into hematopoietic progenitor cells.

In other preferred embodiments, the starting population of cells are mesodermal cells, preferably lateral mesodermal; the mesodermal or lateral cells are differentiated into endothelial cells, preferably arterial endothelial cells; the endothelial or arterial endothelial cells are differentiated into hemogenic endothelial cells, wherein an agents capable of modulating, preferably capable of inhibiting or blocking expression of an Apelin receptor or expression of one or more down-stream activators of Apelin signaling is added to the endothelial cells; optionally the hemogenic endothelial cells are differentiated into hematopoietic progenitor cells.

In other preferred embodiments, the starting population of cells are endothelial cells, preferably arterial endothelial cells; the endothelial or arterial endothelial cells are differentiated into hemogenic endothelial cells, wherein an agents capable of modulating, preferably capable of inhibiting or blocking expression of an Apelin receptor or expression of one or more down-stream activators of Apelin signaling is added to the endothelial cells; optionally the hemogenic endothelial cells are differentiated into hematopoietic progenitor cells.

Preferably, differentiation of ESCs or IPSCs into primitive streak or posterior primitive streak cells is induced by activating BMP, FGF and WNT pathways. Preferably, ESCs or IPSCs are differentiated into primitive streak or posterior primitive streak cells for about 48 hours.

Preferably, differentiation of primitive streak or posterior primitive streak cells into mesodermal or lateral mesodermal cells is induced by activating of BMP, RA, PKA, and VEGF pathways, and inhibiting WNT, TGFβ and PI3K pathways. Preferably, primitive streak or posterior primitive streak cells are differentiated into mesodermal or lateral mesodermal cells for about 24 hours.

Preferably, differentiation of mesodermal or lateral mesodermal cells into endothelial or arterial endothelial cells is induced by activating of TGFβ, VEGF, and RA pathways, and inhibiting BMP, WNT and PI3K pathways. Preferably, mesodermal or lateral mesodermal cells are differentiated into endothelial or arterial endothelial cells for about 24 hours.

Preferably, differentiation of endothelial or arterial endothelial cells into hemogenic endothelial cells is induced by activating GP130, NOTCH, and PKA, pathways, and inhibiting TGFβ and PRC2 pathways. Preferably, differentiation of endothelial or arterial endothelial cells are differentiated into hemogenic endothelial cells for about 72 hours.

Preferably, differentiation of hemogenic endothelial cells into hematopoietic progenitor cells is induced by activating NOTCH and PKA pathways, and inhibiting TGFβ, PRC2, aryl hydrocarbon receptor, LSD1, and G9A/GLP pathways. Preferably, differentiation of hemogenic endothelial cells are differentiated into hematopoietic progenitor cells for about 72 hours.

In one embodiment, said starting cell population is enriched in a desirable cell marker phenotype (e.g., CD34+, CD133+, CD90+) or based on efflux of dyes such as rhodamine or aldehyde dehydrogenase activity. Methods for enriching blood cell population in CD34+ cells include kits commercialized by Miltenyi Biotec (CD34+ direct isolation kit, Miltenyi Biotec, Bergisch, Gladbach, Germany) or by Baxter (Isolex 3000).

In certain embodiments, the cells of the starting population are derived from cells of a patient with a genetic disorder associated with a gene having a sequence defect, and the progenitors of immune and blood cells have been genetically engineered to correct the sequence defect.

In certain embodiments, the method comprises the initial step of harvesting cells for the starting population. Harvesting preferably includes the step of dislodging or separating cells. This can be accomplished using a number of methods, such as enzymatic, non-enzymatic, centrifugal, electrical, or size-based methods, or preferably, flushing the cells using culture media (e.g., media in which cells are incubated) or buffered solution.

The starting population of cells may first be subjected to enrichment or purification step(s), including negative and/or positive selection of cells based on specific cellular markers in order to provide the starting cell population, such as fluorescent activated cell sorting (FACS, flow cytometry) or antibody-based separation methods.

In certain embodiments, the method further comprises the step(s) of collecting and/or, separating, and/or expanding, and/or differentiating the produced progenitors of blood and immune cells . In certain embodiments, the method further comprises the step of inducing the progenitors of blood and immune cells to differentiate into mature blood cells.

### Progenitor cells of blood and immune cells

In preferred embodiments, the progenitors of blood and immune cells are selected from the group consisting of hemogenic endothelial cells (HECs), hematopoietic progenitor cells (HPCs), hematopoietic stem cells (HSCs), and combinations thereof. Preferably, the progenitors of blood and immune cells are hemogenic endothelial cells (HECs) and/or hematopoietic progenitor cells (HPCs).

In preferred embodiments, the progenitor cells of blood and immune cells express transcription factors HLF and/or HOXA, preferably HOXA is HOXA5, HOXA7, HOXA9, and HOXA10. In preferred embodiments, the progenitor cells of blood and immune cells express transcription factors HLF and HOXA, preferably HOXA is HOXA5, HOXA7, HOXA9, and HOXA10.

Preferably, the term "progenitors of blood and immune cells" as used herein includes hemogenic endothelial cells, hematopoietic progenitors and hematopoietic stem cells (HSCs). In preferred embodiments, progenitors of blood and immune cells comprise or consist of hemogenic endothelial cells and hematopoietic progenitors.

Preferably, the term "blood and immune cells" comprises myeloid and lymphoid cells. Myeloid cells include myeloblasts, granulocytes, monocytes, macrophages, neutrophils, basophils, eosinophils, erythrocytes, mast cells, dendritic cells, and megakaryocytes or platelets, and subtypes thereof. Lymphoid cells include lymphocytes, T cells, B cells, natural killer cells, innate lymphoid cells, plasma cells, and subtypes thereof.

Preferably, the blood and immune cells are granulocytes, monocytes, megakaryocytes, fetal hemoglobin-expressing erythroid cells, and macrophages, natural killer (NK) cells, and T cells.

Preferably, the method comprises the additional step of differentiating the hematopoietic progenitor cells into blood and immune cells, preferably into myeloid, erythroid, and/or lymphoid cells, more preferably into granulocytes, monocytes, megakaryocytes, fetal hemoglobin-expressing erythroid cells, macrophages natural killer (NK) cells and/or T cells.

Preferably, the term "hemogenic endothelial cells" relates to a subset of developing vascular endothelial cells that acquire hematopoietic potential and can give rise to multilineage hematopoietic stem and progenitor cells. Lateral mesodermal cells and arterial endothelial cells can give rise to hemogenic endothelial cells in vitro and in vivo. In preferred embodiments, the hemogenic endothelial cells are HOXA+, preferably HOXA5+, HOXA7+, HOXA9+, and HOXA10+. In preferred embodiments, the hemogenic endothelial cells are CD34+, CD144+ and/or RUNX1+. In preferred embodiments, the hemogenic endothelial cells are CD34+, CD144+, RUNX1+ and/or HOXA+, preferably HOXA5+, HOXA7+, HOXA9+, and HOXA10+.

Preferably, the term "hematopoietic progenitor cells" (HPCs) are a population of precursor cells that possess the capacity for self-renewal and multilineage differentiation. HPCs can be differentiated in vitro into various hematopoietic lineages, based on the use of recombinant cytokines, co-culture systems and/or small molecules. In preferred embodiments, the hematopoietic progenitor cells express transcription factors HOXA, preferably HOXA5, HOXA7, HOXA9, and HOXA10. In more preferred embodiments, the hematopoietic progenitor cells express transcription factors HLF and HOXA, preferably HOXA5, HOXA7, HOXA9, and HOXA10. In preferred embodiments, the hematopoietic progenitor cells are CD34+, CD43+, CD45+, CD144+, HLF+ and HOXA+, preferably HOXA5+, HOXA7+, HOXA9+, and HOXA10+.

The term "hematopoietic stem cells" (HSCs) as used herein refer to immature blood cells having the capacity to self-renew and to differentiate into more mature blood cells comprising granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), and monocytes (e.g., monocytes, macrophages). It is known in the art that such cells may or may not include CD34+ cells. CD34+ cells are immature cells that express the CD34 cell surface marker. CD34+ cells are believed to include a subpopulation of cells with the stem cell properties defined above. It is well known in the art that HSCs include pluripotent stem cells, multipotent stem cells (e.g., a lymphoid stem cell), and/or stem cells committed to specific hematopoietic lineages. The stem cells committed to specific hematopoietic lineages may be of T cell lineage, B cell lineage, dendritic cell lineage, Langerhans cell lineage and/or lymphoid tissue-specific macrophage cell lineage. In addition, HSCs also refer to long term HSC (LT-HSC) and short-term HSC (ST-HSC). ST-HSCs are more active and more proliferative than LT-HSCs. However, LT-HSC have unlimited self-renewal (i.e., they survive throughout adulthood), whereas ST-HSC have limited self-renewal (i.e., they survive for only a limited period of time). Any of these HSCs can be used in any of the methods described herein. Optionally, ST-HSCs are useful because they are highly proliferative and thus, quickly increase the number of HSCs and their progeny. Hematopoietic stem cells are optionally obtained from blood products. A blood product includes a product obtained from the body or an organ of the body containing cells of hematopoietic origin. Such sources include un-fractionated bone marrow, umbilical cord, peripheral blood, liver, thymus, lymph and spleen. All of the aforementioned crude or un-fractionated blood products can be enriched for cells having hematopoietic stem cell characteristics in ways known to those of skill in the art.

The term "multipotent" when used in reference to a "multipotent cell" refers to a cell that has the developmental potential to differentiate into cells of one or more germ layers, but not all three. Thus, a multipotent cell can also be termed a "partially differentiated cell." Multipotent cells are well known in the art, and examples of multipotent cells include adult stem cells, such as for example, hematopoietic stem cells and neural stem cells. "Multipotent" indicates that a cell may form many types of cells in a given lineage, but not cells of other lineages. For example, a multipotent hematopoietic cell can form all of the many different types of blood cells (red, white, platelets, etc. . . . ), but it cannot form neurons. Accordingly, the term "multipotency" refers to a state of a cell with a degree of developmental potential that is less than totipotent and pluripotent.

The cell population obtained after the step of culturing may be used without further purification or may be subject to purification or selection steps. The cell population obtained after the step of culturing may be washed to remove the agent and/or any other components of the cell culture and resuspended in an appropriate cell suspension medium for short term use or in a long-term storage medium, for example a medium suitable for cryopreservation.

The term "agent" as used herein refers to a compound or entity of any chemical class including, for example, polypeptides, nucleic acids, saccharides, small molecules, metals, hormones, antibodies, antibody fragments, aptamers, nucleic acids (e.g., siRNAs, shRNAs, antisense oligonucleotides, ribozymes), peptides, peptide mimetics, polymers, etc. or combinations thereof.

The terms "differentiating a cell population in presence of one or more agents" or "culturing a cell population in presence of one or more agents" as used herein includes subjecting a cell population or at least one cell thereof to a culture medium which comprises the one or more agent, for at least one time or a plurality of times. Preferably, the term "differentiating a cell population" includes the process in which a cell changes or is forces to change from one cell type to another one, typically from a less differentiated cell type to a more differentiated cell type. Also included are methods whereby the one or more agents are forced to contact a cell population or at least one cell thereof, for at least one time or a plurality of times. Conditions for culturing the starting cell population for producing and differentiating progenitor cells of blood and immune cells will vary depending, inter alia, on the starting cell population, the desired final number of cells, and desired final proportion of progenitor cells of blood and immune cells. The starting cell population may be used directly for differentiation or frozen and stored for use at a later date.

Preferably, the population of cells is cultured for a period of about 2 days, or 3 days, or 4 days, or 5 days, or 6 days, or 7 days or 8 days or 9 days or 10 days or 12 days or longer. Preferably, the cells are cultured for a period of about 10 days, wherein the starting population are embryonic stem cells or induced pluripotent stem cells (iPSCs).

### Inhibition of Apelin pathway

In preferred embodiments, the endothelial cells and/or progenitor cells thereof, preferably the endothelial cells, are cultured in presence of one or more agents capable of inhibiting or blocking Apelin signaling, capable of inhibiting or blocking expression of an Apelin receptor, and/or capable of inhibiting or blocking expression of one or more down-stream activators of Apelin signaling.

The agents capable of inhibiting or blocking Apelin signaling, capable of inhibiting or blocking expression of Apelin receptor, or capable of inhibiting or blocking expression of one or more down-stream activators of Apelin signaling can be added to the endothelial cells, preferably the arterial endothelial cells.

As used herein the term to "inhibit Apelin signaling" refers to reducing the effect of Apelin, preferably the reduction is measurable. As used herein the term to "block Apelin signaling" refers to preventing the effect of Apelin. The inhibition or blockage of Apelin signaling includes preferably genetic and pharmacological inhibition or blockage of Apelin signaling.

In preferred embodiments, the endothelial cells are cultured in presence of one or more agents capable of inhibiting or blocking Apelin signaling.

The agent capable of inhibiting or blocking Apelin signaling is mentioned herein as antagonist of Apelin signaling. Preferably, the antagonist of Apelin signaling is an antagonist of an Apelin receptor or an antagonist of an Apelin receptor ligand. Preferably, the antagonist is capable of binding to an Apelin receptor or Apelin receptor ligand, thereby antagonizing Apelin signaling.

The term "antagonist" or "antagonize", as used herein, preferably refers to a molecule that binds to the Apelin receptor or Apelin receptor ligand and does not allow to activate the response typically initiated by the active form of the Apelin receptor or ligand. In some cases, antagonists do not diminish the baseline intracellular response in the absence of an agonist. An antagonist does not necessarily have to function as a competitive binding inhibitor, but may work by sequestering an agonist, or indirectly modulating a downstream effect. An antagonist can activate an Apelin receptor, however to a lesser extent than the activation exhibited by a full agonist or ligand of the Apelin receptor.

In certain embodiments, the agent capable of inhibiting or blocking Apelin signaling is selected from the group consisting of an antibody, antibody fragment, small peptide, and small chemical compound. Preferably, the antibody, antibody fragment, small peptide, or small chemical compound antagonizes the Apelin receptor or Apelin receptor ligand.

As used herein, the term Apelin receptor (interchangeable mentioned herein as APLNR or APJ, doi:10.1038/mp.a000304.01, doi:10.1016/0378-1119(93)90495-O; Uniprot: human P35414, mouse Q9WV08) refers to a G protein-coupled receptor that possesses two endogenous ligands, Apelin and Elabela.

As used herein the term Apelin (also mentioned as APLN; encoded in humans by the apln gene Q9ULZ1 and in mouse by Q9R0R4 (uniprot)) refers to a 77-amino acid precursor protein that is hydrolyzed to produce active peptides of different lengths, such as Apelin-36, Apelin-31, Apelin-17, and Apelin13. The hydrolyzed active peptides are also covered by the term Apelin. ELABELA (ELA, Apela, Toddler; doi:10.1124/pr.119.017533) is a peptide that is encoded in humans by the APELA gene (UniProt P0DMC3).

In certain embodiments, the antagonist of Apelin signaling is selected from the group consisting of proteins, peptides, non-peptide and non-protein molecules. In certain embodiments, the antagonist of Apelin signaling includes, but is not limited to antibodies including full length antibodies, antibody fragments, such as single chain antibodies, cameloid antibodies, single domain antibodies (e.g., shark single domain antibodies), single chain or Tandem diabodies, VHH antibodies, anticalins, nanobodies, minibodies, BiTEs, ankyrin, repeat proteins or DARPINs, Avimers, a DART, a TCR-like antibody, Adnectins, Affilins, Transbodies, Affibodies, a TrimerX, MicroProteins, Centyrins, BiCyclic peptides, peptide aptamers, Kunitz domain derived antibody constructs, stapled peptides, antibody-like binding peptidomimetics, antibody-like binding scaffold proteins, monobodies, aptamers and/or other non-antibody proteins scaffold, for example as reviewed in the literature (Vazquez-Lombardi R et al., 2015), small peptides, and small chemical compounds. In certain embodiments, the antagonist of an Apelin receptor is selected from the group consisting of antibodies, and fragments thereof, monobodies, aptamers, affibodies, small peptides, and small chemical compounds. In certain embodiments, the antagonist of Apelin signaling is selected from the group consisting of antibodies, antibody fragments, small peptides, and small chemical compounds.

In certain embodiments, the antagonist of Apelin signaling is selected from the group consisting of antibodies, and fragments thereof, monobodies, and small molecules.

In certain embodiments, the antagonist of Apelin signaling is a small molecule. The term small molecules can include, but is not limited to a peptide, a peptidomimetic, an amino acid, an amino acid analog, a polynucleotide, a polynucleotide analog, an aptamer, a nucleotide, a nucleotide analog, an organic or inorganic compound (e.g., including heterorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

In certain embodiments, the antagonist of Apelin signaling is selected from the group consisting of antibodies, and fragments thereof, monobodies, small peptides, and small chemical compounds. In certain embodiments, the antagonist of Apelin signaling is a small peptide or small chemical compound.

In certain embodiments, the antagonist of Apelin signaling is a small chemical compound. Preferably, the small chemical compound antagonist can be chemically synthetized. Preferably, the small chemical compound antagonist has a low molecular weight, preferably of less than about 1000 Daltons or less than about 500 Daltons. In certain embodiments, the small chemical compound antagonist has a size of about 1 nm.

In certain embodiments, the antagonist of Apelin signaling is a small peptide. Preferably, a small peptide antagonist is a peptide of less than 500, less than 200, less than 100, less than 50 or less than 20 amino acids. In certain embodiments, a small peptide antagonist is a peptide of less than 50 or less than 20 amino acids. In certain embodiments, a small peptide antagonist is a peptide of less than 100 amino acids.

In certain embodiments, the agent capable of inhibiting or blocking Apelin signaling is selected from the group consisting of protamine, protamine fragments and salts thereof; 4-oxo-6-((pyrimidin-2-ylthio)methyl)-4H-pyran-3-yl 4-nitrobenzoate (ML221), ALX 40-4C Trifluoroacetate, ALX 40-4C, ML339, (Ala13)-Apelin-13, Apelin-13 trifluoroacetate; MM54, MM107, MM108, MM193, MM262, MM297, MM298, MM299, MM300, MM301, MM302, MM312, MM313, MM314, MM315, MM316, MM412, MM413, MM414, MM415, MM416, MM417, MM418, MM419, MM420, MM421, MM422, MM423, MM424, MM426, MM428; an anti-APLNR ligand antibody, preferably JN241, JN126, JN126Fc, JN241Fc, JN126FcP13, and an anti-APLNR antagonist antibody, preferably H2aM9232N.

In a preferred embodiment, the antagonist of Apelin signaling is an Apelin receptor blocking or inhibiting peptide. The term "peptide" as used herein refers to any compound produced by amide formation between a carboxyl group of one amino acid and an amino group of another.

In a preferred embodiment, the antagonist of Apelin signaling is selected from the group consisting of protamine (ATC-Code V03AB14), protamines, protamine fragments and salts thereof; 4-oxo-6-((pyrimidin-2-ylthio)methyl)-4H-pyran-3-yl 4-nitrobenzoate (ML221), ALX 40-4C Trifluoroacetate, ALX 40-4C (CAS. Nr. 143413-49-4), ML339 (CAS. Nr. 2579689-83-9), (Ala13)-Apelin-13 (CAS. Nr. 568565-11-7), Apelin-13 trifluoroacetate; MM54 (CAS. Nr. 1313027-43-8), MM107, MM108, MM193, MM262, MM297, MM298, MM299, MM300, MM301, MM302, MM312, MM313, MM314, MM315, MM316, MM412, MM413, MM414, MM415, MM416, MM417, MM418, MM419, MM420, MM421, MM422, MM423, MM424, MM426, and MM428.

In a preferred embodiment, the antagonist of Apelin signaling is a protamine. As used herein the term protamine includes preferably protamine (ATC-Code V03AB14), human protamine 1 (UniProt P04553) and human protamine 2 (UniProt P04554), protamine fragments, preferably protamine 1-4 as disclosed in doi: 10.1096/fj.201601074R and salts thereof, preferably protamine sulfate or hydrochloride, more preferably protamine sulfate.

In a certain embodiment, the antagonist of Apelin signaling is selected from the group consisting of 4-oxo-6-((pyrimidin-2-ylthio)methyl)-4H-pyran-3-yl 4-nitrobenzoate (ML221), MM54 (CAS. Nr. 1313027-43-8), ALX 40-4C Trifluoroacetate, and ALX 40-4C (CAS. Nr.: 143413-49-4), ML339 (CAS. Nr. 2579689-83-9), Apelin-13 trifluoroacetate and (Ala13)-Apelin-13 (CAS. Nr. 568565-11-7). In a certain embodiment, the antagonist of an Apelin receptor is selected from the group disclosed in US20210155659A1 which is incorporated herein in its entirety.

In certain embodiments, the antagonist of Apelin signaling is an antibody or antibody fragment, preferably an antibody. Preferably, antibody or antibody fragment is an antibody antagonist to the Apelin receptor (APLNR) or Apelin receptor ligand, preferably to the human Apelin receptor or Apelin receptor ligand. In certain embodiments, the antagonist of Apelin signaling is a neutralizing, inhibiting or blocking antibody. A neutralizing, inhibiting or blocking antibody, as used herein, refers preferably to an antibody whose binding to APLNR: (i) interferes with the interaction between APLNR or an APLNR fragment and an APLNR receptor ligand, and/or (ii) results in inhibition of at least one biological function of APLNR. The inhibition or blockade caused by an antibody antagonist needs not be complete so long as it is detectable using an appropriate assay.

As used herein, the term "antibody" refers preferably to a polypeptide that includes canonical immunoglobulin sequence elements sufficient to confer specific binding to a particular target, such as an Apelin receptor or Apelin receptor ligand, with the effect to inhibit or block Apelin signaling. The term antibody includes naturally produced antibodies (e.g., generated by an organism reacting to an antigen), or antibodies produced by recombinant engineering, chemical synthesis, or other artificial system, or methodology. The term antibody includes monoclonal, polyclonal or oligoclonal antibodies, preferably monoclonal antibodies. In some embodiments, a provided antibody or antigen-binding fragment thereof may be of an IgG, IgA, IgE, or IgM isotype (preferably human ones), as it can be most appropriate for a given use. In some embodiments, a provided antibody is an IgG isotype, more particularly an IgG1, IgG2, IgG3, or IgG4 isotype. An antibody utilized in accordance with the present invention can be in a format selected from, but not limited to, heteroconjugate antibodies, bi- or multi-specific antibodies (e.g., Zybodies^{®}, elc.), full-length antibodies, antibody fragments as long as they exhibit the desired inhibiting activity, such as single chain variable domains (scFv), polypeptide-Fc fusions, a Fab fragment, a F(ab')2 fragment, cameloid antibodies, heavy-chain shark antibody (IgNAR), scFv-Fc fragment, a single chain, monobodies, a domain antibody, disulfide stabilized Fv proteins ("dsFv"), nanobodies, masked antibodies (e.g., Probodies^{®}), antibody (scAb), chimeric antibodies, recombinant antibodies, especially recombinant human antibodies, antigen-binding fragments of recombinant antibodies, heterologous antibodies, heterohybrid antibodies or antibodies displayed upon the surface of a phage or displayed upon the surface of a cell (e.g., a chimeric antigen receptor T cell). In some embodiments, an antibody or antigen-binding fragment thereof (and particularly a monoclonal antibody), may be a rabbit, mouse, chimeric, humanized or fully human antibody or antigen-binding fragment thereof.

In certain embodiments, the antagonist of Apelin signaling is selected from the group consisting of a monoclonal antibody (mAbs) that bind to the Apelin receptor ligands including Apelin-13, -17, -36, and pyro-Apelin-13 (disclosed doi: 10.1158/1538-7445.AM2018-5756); a single-domain antibodies against human Apelin receptor, such as JN241, JN126, JN126Fc, JN241Fc, and JN126Fc fused to Apelin13, JN126FcP13, (disclosed in doi: 10.1126/sciadv.aax7379 and doi: 10.1038/s42003-020-0867-7, both incorporated in their entirety by reference); and an anti-APLNR antagonist antibody, such as H2aM9232N (disclosed in WO2015077491A1, incorporated in its entirety by reference).

In certain embodiments, the agent capable of inhibiting or blocking expression of an Apelin receptor or expression of one or more down-stream effectors of Apelin signaling is (i) a small interfering RNA molecule, or (ii) an antisense oligonucleotide.

Antisense oligonucleotides and small interfering RNA molecule which can be used to inhibit protein expression are preferably designed in a way that the oligonucleotides and RNA molecules specifically bind the designated mRNA within cells in a way which inhibits translation thereof. Sequences suitable for design and synthesis of antisense oligonucleotides which specifically bind to mRNA, genomic DNA and/or its promoter or other control sequences of Apelin receptors or down-stream effectors of Apelin signaling are known in the art (e.g., doi: 10.3390/ijms21218376). In addition, algorithms for identifying sequences with the highest predicted binding affinity for their target nucleic acid based on thermodynamic cycle that accounts for the energetics of structural alterations in both the target mRNA and the oligonucleotides are also known to the skilled person.

### Cell culture medium

In some embodiments, said starting population of cells and its descendants are cultured in at least one medium. The medium can be liquid, solid and semi-solid media. Preferably, the medium is solid or semi-solid. In certain embodiments, the starting population is seeded as single cells prior to commencing differentiation.

In certain embodiments, the starting population is cultured in a medium comprising serum, such as fetal bovine serum (FBS). In more preferred embodiments, the starting population is cultured in a serum-free medium.

In certain embodiments, the starting population is cultured in at least one medium comprising at least one growth factor.

As used herein, the term "growth factor" is used broadly to encompass factors that modulate the growth, proliferation, survival, differentiation, and/or function of a cell. The growth factor can be any type of molecule, such as a protein or a chemical compound that promotes cellular proliferation and/or survival. The term "growth factor" encompasses cytokines, chemokines, and some hormones. Exposing cells to one or more growth factors can be done prior to, concurrently with, or following exposure of the cells to agents capable of inhibiting or blocking Apelin signaling.

In preferred embodiments, the starting population is cultured in a medium comprising thiazovivin for differentiation of ESCs or iPSCs; or
a medium comprising a bone morphogenetic protein (BMP), preferably BMP4, a WNT activators (such as GSK3 inhibitors, e.g., CHIR99021), and a Fibroblast Growth Factor (FGF) preferably FGF2, for differentiation of posterior primitive streak; or
a medium comprising a bone morphogenetic protein, preferably BMP4, an activators of PKA (e.g., Forskolin), RA (e.g., TTNPB), and VEGF pathways, and inhibitors of TGF-β (e.g., SB505124), WNT (e.g., XAV939), and PI3K (e.g., GDC-0941) pathways, and optionally AA2P, for differentiation of lateral mesodermal cells; or
a medium comprising an activators of TGF-β (e.g., activin A), VEGF, and RA (e.g., TTNPB) pathways, and an inhibitor of BMP (e.g., DMH1), WNT (e.g., XAV939), and PI3K (e.g., GDC-0941) pathways, and optionally AA2P, for differentiation of arterial endothelial cells; or
a medium comprising activators of GP130 (e.g., OSM and LIF), NOTCH (e.g., DLL4-E12), and PKA (e.g., Forskolin) pathways, and an inhibitor of TGF-β (e.g., SB505124) and PRC2 (e.g., UNC1999) pathways, for differentiation of hemogenic endothelial cells; or
a medium comprising an activator of PKA pathways (e.g., Forskolin), and an inhibitor of TGF-β (e.g., SB505124), PRC2 (e.g., UNC1999), G9A/GLP (e.g., UNC0638), aryl hydrocarbon receptor (e.g., SR1), and LSD1 (e.g., UM171) pathways, for differentiation of hematopoietic progenitors.

In certain embodiments, the growth factors present in the medium include one or more of the following growth factors: bone morphogenetic proteins (BMPs), preferably 4 (BMP-4), Fibroblast Growth Factor (FGF), preferably FGF2, vascular endothelial growth factor (VEGF), stem cell factor (SCF), also known as the c-kit ligand or mast cell growth factor, Flt-3 ligand (Flt-3L), iterieukin-6 (IL- 6), interleukin-3 (IL-3), interieukin-7 (IL-7), interleukin-11 (IL-11), thrombopoietin (TPO), granulocyte-macrophage colony stimulating factor (GM-CSF), granulocyte colony stimulating factor (G-CSF), angiopoietin-like proteins (Angptls) (Angptl2, AngptB, AngptlS, Angptl7, and Mfap4), insulin growth factor-2 (IFG-2), IGFBP2, Wnt3a, and fibroblast growth factor-1 (FGF-1).

In more preferred embodiments, the growth factors present in the medium include one or more of the following growth factors bone morphogenetic proteins (BMPs), preferably 4 (BMP-4), Fibroblast Growth Factor 2 (FGF2), vascular endothelial growth factor (VEGF), and a cytokine of interleukin 6 group.

In certain embodiments, the at least one growth factor is bone morphogenetic protein 4 (BMP-4) and/or vascular endothelial growth factor (VEGF). In certain embodiments, the at least one growth factor is BMP-4 and FGF2. n certain embodiments, the at least one growth factor isa cytokine of interleukin 6 group

In certain embodiments, the cells of the starting population are cultured in a first medium comprising bone morphogenetic protein, preferably BMP-4 and/or FGF, preferably FGF2; and the cells of step (i) are subsequently cultured in a second medium comprising BMP, preferably BMP-4 and VEGF.

In certain embodiments, the at least one medium is a first medium comprising BMP, preferably BMP-4 and/or FGF, preferably FGF2, and a second medium comprising at least one growth factor selected from the group consisting of insulin-like growth factor-1 (IGF-1), FMS-like tyrosine kinase ligand (FLT3-L), stem cell factor (SCF), IL-1, IL-3, IL-6, granulocyte colony-stimulating factor (G-CSF), thrombopoietin (TPO) and combinations thereof.

In certain embodiments, the at least one medium is a first medium comprising BMP, preferably BMP-4 and/or vascular endothelial growth factor (VEGF); and a second medium comprising at least one growth factor selected from the group consisting of insulin-like growth factor-1 (IGF-1), FMS-like tyrosine kinase ligand (FLT3-L), stem cell factor (SCF), IL-1, IL-3, IL-6, granulocyte colony-stimulating factor (G-CSF), and thrombopoietin (TPO).

In certain embodiments, the at least one medium is a first medium comprising BMP, preferably BMP-4 and FGF, preferably FGF2; and a second medium comprising BMP, preferably BMP-4, and vascular endothelial growth factor (VEGF); and a third medium comprising a cytokine of interleukin 6 group.

In a further aspect, the invention relates to a kit for producing progenitors of blood and immune cells, comprising one or more agents capable of inhibiting or blocking Apelin signaling, capable of inhibiting or blocking expression of an Apelin receptor and/or capable of inhibiting or blocking expression of one or more down-stream activators of Apelin signaling; and instructions for use in a method of the invention and, optionally, growth factors and/or cell growth media.

Optionally, the kit comprises one or more containers filled with one or more of the agents, growth factors and/or media described herein. Such kits optionally comprise solutions and buffers as needed or desired. The kit optionally includes starting population of cells, said starting population comprises endothelial cells and/or progenitor cells thereof, preferably said starting population comprises arterial endothelial cells and/or progenitor cells thereof or can contain containers or compositions for making progenitor cells of blood and immune cells. The kit may further comprise antibodies for monitoring production of the cells, such as anti-CD34, anti-CD133, anti-CD38, anti-CD45RA and/or anti-Thy1 antibodies.

In a further aspect, the invention relates to a cell population comprising progenitor cells of blood and immune cells, obtainable or obtained by the method of the invention.

Preferably, progenitor cells of blood and immune cells are hemogenic endothelial cells (HECs), hematopoietic progenitor cells (HPCs), hematopoietic stem cells (HSCs), and combinations thereof; more preferably progenitor cells of blood and immune cells are hemogenic endothelial cells (HECs) and/or hematopoietic progenitor cells (HPCs). Preferably, the progenitor cells of blood and immune cells express transcription factors HLF and/or HOXA, i.e. are HLF+ and/or HOXA+, preferably HOXA is HOXA5, HOXA7, HOXA9, and HOXA10.

In preferred embodiments, the hemogenic endothelial cells are HOXA+, preferably HOXA5+, HOXA7+, HOXA9+, and HOXA10+. In preferred embodiments, the hemogenic endothelial cells are CD34+, CD144+ and/or RUNX1+. In preferred embodiments, the hemogenic endothelial cells are CD34+, CD144+, RUNX1+ and HOXA+, preferably HOXA5+, HOXA7+, HOXA9+, and HOXA10+.

In preferred embodiments, the hematopoietic progenitor cells are HOXA+, preferably HOXA5+, HOXA7+, HOXA9+, and HOXA10+. In more preferred embodiments, the hemogenic endothelial cells are HLF+ and HOXA+, preferably HOXA5+, HOXA7+, HOXA9+, and HOXA10+. In preferred embodiments, the hematopoietic progenitor cells are CD34+, CD43+, CD45+, and CD144+. In preferred embodiments, the hematopoietic progenitor cells are CD34+, CD43+, CD45+, CD144+, HLF+ and HOXA+, preferably HOXA5+, HOXA7+, HOXA9+, and HOXA10+.

In preferred embodiments, the hemogenic endothelial cells and/or hematopoietic progenitor cells express one or preferably more than one surface marker associated with populations amenable to long-term transplantation, preferably the surface markers associated with populations amenable to long-term transplantation are selected from the group consisting of CD34, CD38, CD71, CD109, HLA-DR, CD90, CD33, CD13, and CD117.

In some embodiments, the cell population according to the invention is resuspended or included in a pharmaceutically acceptable medium suitable for administration to a mammalian host, thereby providing a composition.

In a further aspect, the invention relates to a composition comprising the cell population of the invention and optionally a pharmaceutically acceptable carrier or medium suitable for administration to a mammalian host.

In certain embodiments, the composition of the invention is appropriate for intravenous infusion. In certain embodiments, the composition comprises at least 10⁴ cells / kg transfused in the patient, preferably between 10⁵ cells / kg and 10⁹ cells / kg.

In a further aspect, the invention relates to the cell population of the invention or the composition of the invention for use in cell transplantation in a mammalian subject, preferably a human patient.

The term "cell population" or "population of cells" refers to eukaryotic mammalian, preferably human, cells isolated from biological sources, for example, blood product or tissues and derived from more than one cell.

Transplanted cells are preferably blood and/or immune cells. Preferably, blood and/or immune cells comprise lymphoid, myeloid, and erythroid cells including T cells, NK cells, and macrophages. In certain embodiments, transplanted cells are hematopoietic progenitor cells.

Cell transplantation may be autologous (the patient's own cells are used), allogeneic (the cells come from a donor) or syngeneic (from an identical twin). The term "allogeneic" as used herein preferably refers to a graft (e.g. hematopoietic cells) which are donated by an individual whose genetic characteristics differ from those of the recipient, especially regarding the major histocompatibility complex (MHC) and minor histocompatibility antigens expressed on the surface of the individual's cells. The term "autologous" as used herein refers preferably to a graft (e.g. hematopoietic cells present in the bone marrow or peripheral blood) that uses the subject's own cells. The cells are usually harvested in advance of the subject undergoing treatment.

The subject referred to herein is, for example, an individual with or at risk for depleted or limited blood cell levels, dysfunctional or depleted bone marrow. The subject is optionally a recipient of a bone marrow transplant. The methods and uses described herein are particularly useful in subjects that have limited bone marrow reserve such as elderly subjects or subjects previously exposed to an immune depleting treatment or myeloablative treatment such as chemotherapy, e.g., for treating leukemia or lymphomas. The subject, optionally, has a decreased blood cell level or is at risk for developing a decreased blood cell level as compared to a control blood cell level. As used herein the term control blood cell level refers to an average level of blood cells in a subject prior to or in the substantial absence of an event that changes blood cell levels in the subject. An event that changes blood cell levels in a subject includes, for example, anemia, trauma, chemotherapy, bone marrow transplant and radiation therapy. For example, the subject has anemia or blood loss due to, for example, trauma.

The produced blood and immune cells, or hemogenic endothelial cells and/or hematopoietic progenitor cells or the composition comprising these cells are administered to the subject, for example, before, at the same time, or after chemotherapy, radiation therapy or a bone marrow transplant. The subject optionally has depleted bone marrow related to, for example, congenital, genetic or acquired syndrome characterized by bone marrow loss or depleted bone marrow. Thus, the subject is preferably a subject in need of hematopoiesis. Optionally, the subject is a bone marrow donor or is a subject with or at risk for depleted bone marrow or in need of additional blood or immune cells.

In a preferred embodiment, the cell population of the invention or the composition of the invention comprising said cell population is used for treating a genetic disorder in a patient in need thereof, comprising administering to the patient the cell population of the invention or the composition of the invention, wherein the progenitors of blood and immune cells have been genetically engineered to correct the genetic disorder.

In a preferred embodiment, the cell population of the invention or the composition of the invention is used for treating a patient suffering from pancytopenia or neutropenia, wherein preferably blood, immune cells and/or their progenitor cells thereof are administered to a patient. Preferably, the pancytopenia or neutropenia is caused by chemotherapy, myeloablation, or exposure to radiation.

In a preferred embodiment, the cell population of the invention or the composition of the invention is used for treating a patient suffering from a disease selected from the group consisting of malignancies, hematopoietic disorder, hematologic disease, metabolic disorders, environmentally induced diseases, preferably radiation poisoning, chemotherapy; viral diseases, preferably HTLV, HIV; autoimmune diseases, lysosomal storage disorders, immunodeficiencies preferably inherited immunodeficiencies, and hereditary skeletal dysplasia.

In a preferred embodiment, the cell population of the invention or the composition of the invention is used for treating impaired hematopoiesis.

In a preferred embodiment, the cell population of the invention or the composition of the invention is used for treating a patient suffering from a hematopoietic disorder. In a preferred embodiment, the invention relates to a method for treating a hematopoietic disorder, wherein the method comprises administering to a patient an effective amount of the cell population of the invention or the composition of the invention. Said hematopoietic disorder is preferably selected from the group consisting of acute myeloid leukemia, acute lymphoblastic leukemia, chronic myeloid leukemia, chronic lymphocytic leukemia, myeloproliferative disorders, myelodysplastic syndromes, multiple myeloma, non-Hodgkin lymphoma, Hodgkin disease, aplastic anemia, pure red cell aplasia, paroxysmal nocturnal hemoglobinuria, fanconi anemi, Thalassemia major, sickle cell anemia, severe combined immunodeficiency, Wiskott-Aldrich syndrome, and hemophagocytic lymphohistiocytosis.

In a preferred embodiment, the cell population of the invention or the composition of the invention is used for treating a patient suffering from a malignant disorder, wherein said malignant disorder includes hematological malignancies and/or solid cancer tumors. In a preferred embodiment, the invention relates to a method for treating a malignant disorder, the method comprises administering to a patient an effective amount of the cell population of the invention or the composition of the invention. Preferably, said malignant disorder includes hematological malignancies and/or solid cancer tumors.

Hematological malignancies are preferably selected from the group consisting of leukemias, including acute lymphoblastic leukemia (ALL), acute myeloid leukemia (AML), chronic lymphocytic leukemia (CLL) and chronic myelogenous leukemia (CML), accelerated phase or blast crisis; lymphomas, including Hodgkin's disease, non-Hodgkin's lymphoma; myelomas, such as multiple myeloma (Kahler's disease), malignant infantile osteopetrosis.

Solid cancer tumors are preferably selected from the group consisting of neuroblastoma, germ cell tumors, desmoplastic small round cell tumor, Ewing's sarcoma, and choriocarcinoma

In a preferred embodiment, the cell population of the invention or the composition of the invention is used for treating a patient suffering from a hematologic disease, said hematologic disease is preferably selected from the group consisting of phagocyte disorders, such as myelodysplasia; anemias, preferably paroxysmal nocturnal hemoglobinuria (PNH; severe aplasia), aplastic anemia, acquired pure red cell aplasia; and myeloproliferative disorders, preferably polycythemia vera, essential thrombocytosis, myelofibrosis; hemoglobinopathies, sickle cell disease, β thalassemia major, cytopenias, preferably amegakaryocytic thrombocytopenia; hemophagocytic syndromes, preferably hemophagocytic lymphohistiocytosis.

In a preferred embodiment, the cell population of the invention or the composition of the invention is used for treating a patient suffering from an autoimmune disorder, said autoimmune disorders is preferably selected from the group consisting of multiple sclerosis, systemic lupus erythematosus and systemic sclerosis.

In a preferred embodiment, the cell population of the invention or the composition of the invention is used for treating a patient suffering from an immunodeficiency, said immunodeficiency is preferably congenital immunodeficiencies or acquired immunodeficiency syndrome.

In a preferred embodiment, the cell population of the invention or the composition of the invention is used for treating a patient suffering from an immunodeficiency, said immunodeficiencies is preferably selected from the group consisting of T-cell deficiencies, such as ataxia-telangiectasia, DiGeorge syndrome; combined T- and B-cell deficiencies, such as severe combined immunodeficiency (SCID); Wiskott-Aldrich syndrome; phagocyte disorders, such as Kostmann syndrome, Shwachman-Diamond syndrome; immune dysregulation diseases, such as Griscelli syndrome, type II; innate immune deficiencies, such as NF-Kappa-B Essential Modulator (NEMO) deficiency (Inhibitor of Kappa Light Polypeptide Gene Enhancer in B Cells Gamma Kinase deficiency)

In a preferred embodiment, the cell population of the invention or the composition of the invention is used for treating a patient suffering from a metabolic disease, said metabolic disease is preferably selected from the group consisting of amyloidosis, preferably amyloid light chain amyloidosis; inborn errors of metabolism, lysosomal storage disorders, preferably Lipidoses including neuronal ceroid lipofuscinoses, infantile neuronal ceroid lipofuscinosis, Jansky-Bielschowsky disease (late infantile neuronal ceroid lipofuscinosis), sphingolipidoses, Niemann-Pick disease, Gaucher disease, leukodystrophies, adrenoleukodystrophy, metachromatic leukodystrophy, and Krabbe disease (globoid cell leukodystrophy).

In certain embodiments, the inborn errors of metabolism are selected from mucopolysaccharidosis, Gaucher disease, metachromatic leukodystrophies and adrenoleukodystrophies.

In a preferred embodiment, the invention relates to a method for treating the above-mentioned diseases or disorders, wherein the method comprises administering to a patient an effective amount of the cell population of the invention or the composition of the invention.

Preferably, the one or more agents capable of inhibiting or blocking Apelin signaling, capable of inhibiting or blocking expression of an Apelin receptor and/or capable of inhibiting or blocking expression of one or more down-stream activator of Apelin signaling are present in amounts that are sufficient to produce progenitor cells of blood and immune cells.

In a further aspect, the invention relates to a biological material comprising blood and immune cells or progenitors thereof produced by the method of the invention.

In a further aspect, the invention relates to a pharmaceutical composition comprising one or more agents capable of inhibiting or blocking Apelin signaling and a population of progenitor cells of blood and immune cells. Preferably, the progenitor cells of blood and immune cells are endothelial cells, more preferably arterial endothelial cells. Said pharmaceutical composition is preferably for producing blood and immune cells or descendants of endothelial cells.

Another aspect of the invention relates to use of one or more agents that inhibit or block Apelin signaling for production of blood and immune cells or progenitors thereof.

The term "expansion" in the context of cells refers to increase in the number of a characteristic cell type, or cell types, from an initial cell population of cells, which may or may not be identical. The initial cells used for expansion may not be the same as the cells generated from expansion.

The term "graft" as used herein refers to a biological sample selected from bone marrow, blood (e.g. whole blood or peripheral blood mononuclear cells (PBMCs), blood products, or solid organs in which hematopoietic cells are present.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. An individual is successfully "treated", for example, if one or more symptoms associated with a disease are mitigated or eliminated.

The term "subject" as used herein refers to a mammal including human and non-human animals. More particularly, the mammal is a human. Terms such as "subject", "patient" or "individual" are terms that can, in context, be used interchangeably in the present disclosure. In certain examples, the subject may be an adult or a child (pediatric) subject. A subject can be one who has been previously diagnosed with or identified as suffering from or having a disease or disorder in need of treatment or one or more complications related to such a disease or disorder, and optionally, have already undergone treatment for the disease or disorder or the one or more complications related to the disease or disorder. Alternatively, a subject can also be one who has not been previously diagnosed as having such a disease or disorder (e.g., decreased blood cell level) or related complications. For example, a subject can be one who exhibits one or more risk factors for the disease or disorder or one or more complications related to the disease or disorder or a subject who does not exhibit risk factors. The terms "a patient suffering from" or "a patient having" a certain disease are interchangeably used herein.

An "effective amount" refers to at least an amount effective, at dosages and for periods of time necessary, to achieve the desired therapeutic or prophylactic result. An effective amount can be provided in one or more administrations. In some examples of the present disclosure, the term "effective amount" is used to refer to an amount necessary to effect treatment of a disease or condition as hereinbefore described. The effective amount may vary according to the disease or condition to be treated and also according to the weight, age, racial background, sex, health and/or physical condition and other factors relevant to the mammal being treated. Typically, the effective amount will fall within a relatively broad range (e.g. a "dosage" range) that can be determined through routine trial and experimentation by a medical practitioner. The effective amount can be administered in a single dose or in a dose repeated once or several times over a treatment period.

The terms "pharmaceutical" and "pharmaceutically acceptable" are employed herein to refer to those carriers, media and compounds, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent or encapsulating material, involved in carrying or transporting the agents from one organ, or portion of the body, to another organ, or portion of the body. Each carrier must be "acceptable" in the sense of being compatible with the other ingredients of the formulation, for example the carrier does not decrease the impact of the agent on the treatment. In one embodiment, a carrier is pharmaceutically inert. The terms "physiologically tolerable carriers" and "biocompatible delivery vehicles" are used interchangeably.

Unless otherwise indicated, the stem cells, cell culture, surgical techniques, and all technical and scientific terms utilized in the present disclosure are standard procedures, well known to those skilled in the art. Such techniques are described and explained throughout the literature in sources such as Perbal, 1984; Sambrook & Green, 2012; Brown, 1991; Glover & Hames, 1995 and 1996; Ausubel et al., Current Protocols in Molecular Biology, Greene Publishing Associates (1992), including all updates until present; and Harlow and Lane Antibodies: A Laboratory Manual, Cold Spring Harbor Laboratory Press, Cold Spring Harbor, N.Y. (1990), including all updates until present; and Coligan et al., 1991 including all updates until present. Although methods and materials similar or equivalent to those described herein can be used in the practice or testing of the present invention, suitable methods and materials are described below. In case of conflict, the present specification, including definitions, will control.

While the invention is illustrated and described in detail in the drawings, examples and description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Throughout this specification and the claims, unless the context requires otherwise, the word "comprise" or "include", and variations such as "comprises/includes" and "comprising/including" are to be understood to imply the inclusion of an element, stated integer, step or a group thereof but not the exclusion of any other element, stated integer, step or a group thereof.

As used in this specification and the appended claims, the singular forms "a", "an", and "the" include plural referents, unless the content clearly dictates otherwise.

The term "essentially", "about" or "approximately" and the like when used in connection with an attribute or numerical value particularly defines exactly the attribute or value and additionally encompass, unless the context indicates otherwise, numerical values within a range having a lower limit that is 0-10% smaller than the indicated numerical value and having an upper limit that is 0-10% larger than the indicated numerical value. The term "about" or "approximately" means preferably ±10%, more preferably ±5%, again more preferably ±3% or most preferably ±0% (referring to the given numeric value, respectively). In each of the invention embodiments, "about" can be deleted. All ranges of values disclosed herein, should refer and include to any and all values falling within said range including the values defining the range.

### EXAMPLES

### EXAMPLE 1 - Apelin signaling restricts the formation of the blood stem cell-forming hemogenic endothelium

### Material and Methods

### Zebrafish husbandry and strains

All zebrafish housing and husbandry were performed under standard conditions in accordance with institutional (Philipps-Universität Marburg) and national ethical and animal welfare guidelines approved by the ethics committee for animal experiments at the Regierungspräsidium Gießen, Germany, as well as the FELASA guidelines (Aleström et al., 2020). Embryos were staged by hours post fertilization (hpf) at 28.5°C (Kimmel et al., 1995).

**Table 1: zebrafish lines used in this study**

| **Zebrafish lines** | **Reference** |
|---|---|
| *apln^{mu267}* | (Helker et al., 2015) |
| *aplnrb^{mu281}* | (Helker et al., 2020) |
| *Tg^{BAC}(aplnrb:Venus-PEST)^{mr13}* | (Qi et al., 2022) |
| *Tg(kdrl:NLS-mCherry)^{is4}* | (Wang et al., 2010) |
| *Tg(kdrl:HsHRAS-mCherry)^{s896}* | (Chi et al., 2008) |
| *Tg(-6.0itga2b:GFP)^{la2}* | (Traver et al., 2003) |
| *Tg(hsp70l:apln)^{mu269}* | (Helker et al., 2020) |
| *Tg(kdrl:GFP)^{s843}* | (Beis et al., 2005) |
| *Tg(fli1a:GFP)^{y1}* | (Lawson and Weinstein, 2002) |

### FACS sorting and Quantitative RT-PCR

The AGM region of 120 double transgenic embryos (*Tg^{BAC}(aplnrb:Venus-PEST)^{mr13}*, *Tg(kdrl:NLS-mCherry)^{is4}*) was manually dissected at 48 hpf and digested with TrypLE Express (gibco) for 30 min (samples were mixed by pipetting every 5 min). Reaction was stopped by adding FBS and cell suspension was pelleted by centrifugation, resuspended in ice-cold HBSS + 5% FBS and poured through 40µm filters. Centrifugation, resuspension and filtering were repeated once. Cells were FACS sorted for transgenic expression using a BD FACS Aria III (BD Biosciences). Total RNA was isolated using the miRNeasy Micro Kit (Qiagen) and cDNA was synthesized using the iScript^{™} cDNA Synthesis Kit (Bio Rad). qRT-PCR reaction was performed using an ECO48 real-time qPCR system (PCRmax) with the oligonucleotides listed in Table 2.

**Table 2: oligonucleotides used in quantitative RT-PCR**

| **Oligonucleotides** | **sequence** |
|---|---|
| *rp113 se* | AATTGTGGTGGTGAGGTG |
| *rpl13 as* | GGTTGGTGTTCATTCTCTTG |
| *runx1 se* | CGTCTTCACAAACCCTCCTCAA |
| *runx1 as* | GCTTTACTGCTTCATCCGGCT |
| *aplnrb se* | CCTCTTGCGCTATGGACTTC |
| *aplnrb as* | GCCTGCAATCCAGTAGGTCT |

### Morpholino injections

The *tnnt2a*MO1 morpholino (Sehnert et al., 2002) was obtained from Gene Tools, dissolved in H₂O and 2 ng/embryo were injected into 1 cell stage embryos. An equal amount of the standard MO: 5'-CCTCTTACCTCAGTTACAATTTATA-3' was injected as control.

### Crispant injections

Synthetic sgRNAs were obtained from IDT (Coralville, USA). P0 knockouts were done as described (Quick et al., 2021).

**Table 3: crRNAs**

| **crRNA Name** | **Location** | **Target sequence [PAM]** |
|---|---|---|
| *apln crRNA 1* | Exon 1 | CTATGCTCGGTGGAGGCCAT [TGG] |
| *apln crRNA 2* | Exon 2 | GAATGTGAAGATCTTGACGC [TGG] |
| *apln crRNA 3* | Exon 2 | GAAGCATGAGGACTCCTTTG [CGG] |

### Confocal microscopy

Zebrafish embryos and larvae were raised to the desired developmental stage and mounted in a glass bottom dish in 0.3% agarose containing 20 mg/l Tricaine. Images and time-lapse movies were acquired using an upright Leica ST8 equipped with a tempered chamber.

### Quantification of HSPCs in the DA and CHT

HSCs in the VDA were quantified within a segment spanning five somites. HSCs in the CHT were counted within a segment spanning six somites. Quantification was done by use of the Imaris software (Oxford instruments).

### Whole mount in situ hybridization

Whole mount *in situ* hybridizations were performed as described (Thisse and Thisse, 2008) using previously published probes (Lundin et al., 2020). Results were documented using a SMZ 18 binocular (Nikon) with a DS-Fi3 camera (Nikon). *runx1* and *cmyb* stainings were phenotypically scored for low, medium and high expression and quantification is displayed in a distribution plot.

### EdU cell proliferation assay

Zebrafish embryos were raised in E3 containing 0.1% PTU until 34 hpf, dechorionated and transferred into 500 µM EdU solution with 10% DMSO in E3. Embryos were incubated in the EdU solution for 1 h on ice and were then allowed to recover for 1 h at 28.5°C in E3 and fixed in 4% PFA for 1 h at room temperature. EdU labelling was performed according to manufacturer instructions (Click-iT^{™} Plus EdU Cell Proliferation Kit, Alexa Fluor^{™} 647 dye, Invitrogen #C10640). Images were recorded with an upright Leica ST8 confocal microscope. Statistical analysis: Statistical analysis of the acquired data was performed in Prism 9 (GraphPad).

### Results

### Apelin receptor b (aplnrb) negative hemogenic endothelial cells transition into hematopoietic stem cells

In order to analyze the expression of the *aplnrb* in the vasculature, the inventors genetically labeled *aplnrb* expressing cells using the *Tg^{BAC}(aplnrb:Venus-PEST)* and the nucleus of endothelial cells with mCherry using the *Tg(kdrl.NLS-mCherry)* zebrafish line. At 20 hours post-fertilization (hpf) the inventors observed ap/nrb:Venus-PEST expression in all ECs within the DA (Fig. 1 A). To our surprise, starting at 22 hpf and more clearly at 24 hpf (Fig. 1 B), a subpopulation of ECs within the VDA lost expression of *aplnrb:*Venus-PEST. At 48 hpf, *aplnrb:*Venus-PEST negative ECs are still present in the VDA (Figure 1 C, D). In order to exclude labelling of non-EC cells by the *Tg(kdrl:NLS-mCherry)* transgenic line, the inventors analyzed *Tg^{BAC}(aplnrb:Venus-PEST); Tg(kdrl:HsHRAS-mCherry)* embryos.

Based on their location in the VDA the inventors speculated that ap/nrb:Venus-PEST negative ECs represent HECs/HSCs. To determine if the *aplnrb* negative cells are HECs/HSCs, the inventors analyzed their transcriptome. Therefore, the inventors FACS sorted ECs within the trunk of 48 hpf old zebrafish larvae based on *aplnrb:*Venus-PEST expression (Figure 1 E) and performed quantitative RT-PCR analysis. Our quantitative RT-PCR revealed that the *aplnrb* negative ECs in the ventral floor of the DA exhibit elevated mRNA levels of the HE/HSC marker gene *runx1* (Figure 1 F), while showing decreased expression of *aplnrb* mRNA levels (Figure 1 G). Furthermore, the inventors could show by quantitative RT-PCR for the vascular EC marker *kdrl* that the *aplnrb* negative ECs in the ventral floor of the DA are losing their vascular identity while acquiring HE properties (Figure 1 F). To verify if the *aplnrb* negative cells in the VDA represent HE/HSC, the inventors performed confocal imaging of the *Tg^{BAC}(ap*/*nrb:Venus-PEST)* line with the *Tg(itga2b:GFP)* line (also known as *Tg(CD41:GFP))* a well-known HSC marker. The inventors observed that the *aplnrb* negative cells in the VDA do express the transgenic HSC reporter *itga2b:GFP* (Figure 1 H-I'). Next, the inventors investigated the behavior of the *aplnrb* negative cells in the VDA by confocal time-lapse imaging (Figure 1 J-J‴). The inventors observed that the *aplnrb* negative cells in the VDA position ventrally, emerge from the DA into the subaortic space and subsequently enter circulation through the PCV (Figure 1 J-J‴) as it has been described for HSC emergence. Together, these results indicate that *aplnrb* negative ECs in the DA represent HECs/HSCs.

### Loss of Apelin signaling results in elevated HSC numbers in the VDA

Since the inventors found that *aplnrb* negative ECs are HECs, the inventors wanted to investigate the function of Apelin signaling during hematopoiesis. To visualize HECs/HSCs, the inventors performed confocal imaging of the transgenic zebrafish line *Tg(itga2b:GFP).* The inventors observed an increased number of HSCs in the DA in maternal-zygotic *apln* (*MZapln*) mutants at 48 hpf (Figure 2 A-B', E). To analyze which receptor is mediating this effect in the DA, the inventors also analyzed zebrafish embryos mutant for the *Apelin receptor b (aplnrb).* However, homozygous *aplnrb* -/mutants display a cardiac defect and lack of blood flow (Deshwar et al., 2016; Qi et al., 2022; Scott et al., 2007; Zeng et al., 2007) which is known to be a regulator of HSC maintenance and emergence (Campinho et al., 2020; Lancino et al., 2018; Lundin et al., 2020; North et al., 2009; Poullet et al., 2019). To exclude that possible differences in HSC numbers in *aplnrb* mutants and siblings are due to differences in blood flow the inventors eliminated cardiac function by injecting a morpholino (MOs) targeting *tnnt2a* (Sehnert et al., 2002), a gene encoding the contractile protein cardiac troponin, into one-cell stage embryos. The inventors observed that loss of *aplnrb* leads to increased HSC numbers in the VDA compared with sibling larvae independent of blood flow (Figure 2 C-D', F). In zebrafish, *runx1* expression marks HECs in the DA from 23 hpf onwards (Wilkinson et al., 2009). *runx1* expression is increased in *MZapln* and *aplnrb* mutants at 24 hpf (Figure 2 G-L). Altogether, this data suggests that Apelin signaling specifically restricts HE development in the DA.

### Elevated HSC numbers are caused by increased transition to hemogenic endothelium in Apelin-deficient larvae

To determine if increased proliferation of HECs/HSCs in the VDA is causing the elevated HSC numbers upon loss of Apelin signaling the inventors performed EdU labelling assays. The number of EdU positive, proliferating cells showed no significant difference in the VDA in *aplnrb* mutant embryos and control siblings at 36 hpf (Figure 3 A-C). The inventors speculated that Apelin signaling restricts a HE fate in arterial ECs and therefore, loss of Apelin signaling leads to an expansion of the HE. To test this hypothesis the inventors employed a genetic approach by injecting three crRNAs targeting different exons (Kroll et al., 2021; Quick et al., 2021) of the *apln* gene into *Tg^{BAC}(aplnrb:Venus-PEST); Tg(gata2b:KaltA4); Tg(UAS:lifeact-GFP)* embryos and analyzed the F0 Crispants at 48 hpf (Figure 3 D-G). Embryos injected with crRNAs targeting *apln* exhibited an increased number of HSCs in the VDA (Figure 3 D-F) consistent with our previous results. Most strikingly, the inventors could observe HSCs that are also expressing *aplnrb* upon loss of Apelin signaling, indicating a transition of vascular ECs to HECs (Figure 3 D-E', G). These results show an extension of the HE at the expense of vascular ECs upon loss of Apelin signaling.

### Expanded hematopoietic stem cell pool of apln mutants can colonize the CHT stem cell niche

Upon emergence from the DA, HSCs enter the circulation and colonize their subsequent niche, the CHT (Murayama et al., 2006). In order to analyze if elevated HSC numbers in *apln* mutants, can subsequently colonize the hematopoietic stem cell niche, the inventors analyzed *Tg(kdrl:HsHRAS-mCherry); Tg(itga2b:GFP)* double transgenic zebrafish larvae by confocal microscopy at 72 hpf. Similar to the HSC number in the DA, the inventors observed a significant increase in the number of HSCs in the niche of *MZapln* mutants compared to wildtype larvae (Figure 4 A-C). The inventors confirmed this data by WISH for *myb,* a well-known marker for HSCs/HSPCs, at 72 hpf (Figure 4 D-E) and 120 hpf (Figure 4 F-G). Hence, the inventors could show that the Apelin deficient HSCs are viable and can colonize their stem cell niche.

### EXAMPLE 2 - HSCs produced for clinical applications - Differentiation of stem cells towards hematopoietic stem cells and addition of Apelin inhibitors

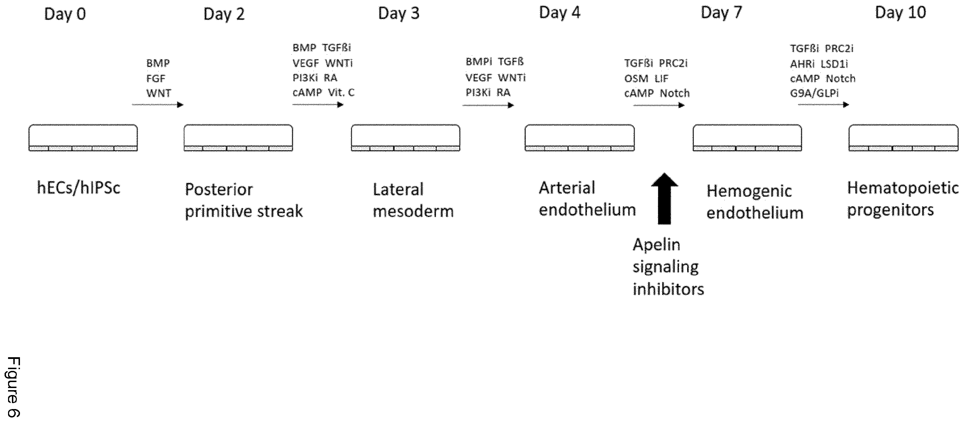

Human pluripotent stem cells (hPSCs) were differentiated into hematopoietic progenitors (Jonas L. Fowler et al., Dev Cell (2024)) and progenitors of hemogenic endothelial cells, such as endothelial cells are treated with one or more Apelin inhibitors (Figure 6).

In brief, undifferentiated hPSCs were dissociated using Accutase (Thermo Fisher, 00-4555-56) and sparsely seeded as single cells prior to commencing differentiation. To reiterate, undifferentiated hPSCs were maintained by passaging them as small clumps (using EDTA-based dissociation, to maintain normal karyotype) but were seeded for differentiation as single cells (using Accutase-based dissociation; to enable efficient differentiation). The initial steps of differentiation (posterior primitive streak, lateral mesoderm, and artery endothelium induction) were conducted in CDM2 basal media.

Seeding human embryonic stem cells (hESCs) or human induced pluripotent stem cells (hIPSs) for differentiation (Step 0): Cells are plated into precoated Geltrex plates in mTeSR supplemented with thiazovivin (1 mM, a ROCK inhibitor [Tocris, 3845]). Freshly-seeded hPSCs or hiPSs were allowed to adhere and recover for 24 hours in mTeSR + 1 mM thiazovivin prior to initiating differentiation.

In detail, the protocol is as follows: Day 1-2 (posterior primitive streak induction, 48 hours) (Step 1): hPSCs or hiPSs were briefly washed (DMEM/F12 [Thermo Fisher, 10565042]) to remove all traces of mTeSR + thiazovivin. Then, they were differentiated towards posterior primitive streak in CDM2 media supplemented with BMP4 (40 ng/mL [R&D Systems, 314-BP-050]), CHIR99021 (6 mM [Tocris, 4423]), and FGF2 (20 ng/mL [R&D Systems, 233-FB-01M]) for 48 hours. Posterior primitive streak induction media was refreshed every 24 hours.

Day 3 (lateralmesoderminduction, 24 hours) (Step2): Day2 posterior primitive streak cells were briefly washed (DMEM/F12) and then differentiated towards lateral mesoderm in CDM2 media supplemented with BMP4 (40 ng/mL), GDC-0941 (2.5 mM [Cellagen Technology, C4321-25]), Forskolin (10 mM [Tocris, 1099]), SB505124 (2 mM [Tocris, 3263]), VEGF (100 ng/mL [R&D Systems, 293-VE-0500]), XAV939 (1 mM [Tocris, 3748]), AA2P (200 mg/mL [Sigma, 49752-10G]), and TTNPB (0.5 nM [Tocris, 0761]) for 24 hours.

Day 4 (artery endothelium induction, 24 hours) (Step3): Lateral mesoderm cells of day 3 were briefly washed (DMEM/F12) and then differentiated towards artery endothelial cells in CDM2 media supplemented with Activin A (15 ng/mL [R&D Systems, 338-AC-500/CF]), DMH1 (250 nM [Tocris, 4126]), GDC-0941 (2.5 mM), VEGF (100 ng/mL), XAV939 (1 mM), AA2P (200 mg/mL), and TTNPB (0.5 nM) for 24 hours.

Subsequently, hPSC-derived day 4 artery endothelial cells were dissociated into single cells (using Accutase), counted, and then re-seeded at high density (500,000 cells/cm²; i.e., 1×10⁶ cells/well of a 24-well plate) on plates that had been coated with 10 mg/mL vitronectin (Thermo Fisher, A14700) + 20 nM of the high-affinity NOTCH agonist DLL4-E12 (from Vincent Luca's laboratory). 300-350 mL of vitronectin/E12 solution was used to coat each well of a 24-well plate, using the method described in a pre- ceding section. At this stage, high cellular seeding density was critical to subsequently achieve efficient blood differentiation. The next steps of differentiation are conducted in CDM3 basal media.

Day 5-7 (hemogenic endothelium induction, 72 hours) (Step4): Addition of Apelin signaling inhibitors.

Artery endothelial cells of day 4 are dissociated into a single-cell suspension (Accutase). Apelin signaling inhibitor is added. Cells densely re-seeded onto plates precoated with 10 mg/mL Vitronectin + 20 nM of high-affinity NOTCH agonist DLL4-E12; and then further differentiated towards hemogenic endothelium in CDM3 media supplemented with Forskolin (10 mM), LIF (20 ng/mL [R&D Systems, 7734-LF-025]), OSM (10 ng/mL [R&D Systems, 295-OM-010]), SB505124 (2 mM), and UNC1999 (1 mM [Tocris, 4904]) for 72 hours. Hemogenic endothelium induction media was refreshed every 24 hours with a complete media change.

Day 8-10 (hematopoietic progenitor induction, 72hours) (Step5): Day 7 hemogenic endothelium cells were differentiated towards hematopoietic progenitors in CDM3 media supplemented with Forskolin (10 mM), SB505124 (2 mM), SR1 (750 nM [Cellagen, C7710-5]) and UM171 (75 nM [ApexBio, A8950]), UNC0638 (500 nM [Tocris, 4343]), and UNC1999 (1 mM) for 72 hours. No washing was performed when adding media to avoid disturbing the emerging semi-adherent cells. Hematopoietic progenitor induction media was refreshed every 24 hours with a complete media change for day 8 and 9, but on the last day (day 10), media was supplemented only.

Day 10 HLF+ HOXA+ hematopoietic progenitors can be used to generate all major types of blood and immune cell in vitro including myeloid, erythroid, and lymphoid cells. Differentiated HLF+ HOXA+ hematopoietic progenitors could be used for engraftment in order to yield myeloid cells in vivo.

## Claims

**1.** A method for producing progenitor cells of blood and immune cells, said method comprises
(i) providing a starting population of cells, said starting population comprises endothelial cells and/or progenitor cells thereof, preferably said starting population comprises arterial endothelial cells and/or progenitor cells thereof; and
(ii) differentiating said starting population into progenitor cells of blood and immune cells,
wherein the endothelial cells and/or progenitor cells thereof are cultured in presence of one or more agents capable of inhibiting or blocking Apelin signaling, capable of inhibiting or blocking expression of an Apelin receptor and/or capable of inhibiting or blocking expression of one or more down-stream activators of Apelin signaling.

**2.** The method of claim 1, wherein the progenitor cells of endothelial cells or arterial endothelial cells are selected from the group consisting of pluripotent stem cell, comprising embryonic stem cells and induced pluripotent stem cell (iPSC); mesodermal cells, preferably lateral mesodermal cells; primitive streak cells, preferably posterior primitive streak cells; hemangioblasts; and combinations thereof.

**3.** The method of any one of the preceding claims, wherein the cells of the starting population express HOXA, preferably HOXA1 to HOXA10, more preferably HOXA1, HOXA3, and HOXA5 to HOXA10.

**4.** The method of any one of the preceding claims, wherein the progenitors of blood and immune cells are selected from the group consisting of hemogenic endothelial cells (HECs), hematopoietic progenitor cells (HPCs), hematopoietic stem cells (HSCs) and combinations thereof, preferably the progenitors of blood and immune cells are hemogenic endothelial cells (HECs) and/or hematopoietic progenitor cells (HPCs).

**5.** The method of any one of the preceding claims, wherein the progenitor cells of blood and immune cells express transcription factors HLF and/or HOXA, preferably HOXA is HOXA5, HOXA7, HOXA9, and HOXA10.

**6.** The method of any one of the preceding claims, wherein the one or more agent(s) capable of inhibiting or blocking Apelin signaling is/are antagonist(s) of an Apelin receptor or an antagonist of an Apelin receptor ligand.

**7.** The method of any one of the preceding claims, wherein the one or more agent(s) capable of inhibiting or blocking Apelin signaling is/are selected from the group consisting of an antibody, antibody fragment, small peptide, and small chemical compound.

**8.** The method of any one of the preceding claims, wherein the one or more agent(s) capable of inhibiting or blocking Apelin signaling is/are selected from the group consisting of protamine, protamine fragments and salts thereof; 4-oxo-6-((pyrimidin-2-ylthio)methyl)-4H-pyran-3-yl 4-nitrobenzoate (ML221), ALX 40-4C Trifluoroacetate, ALX 40-4C, ML339, (Ala13)-Apelin-13, Apelin-13 trifluoroacetate; MM54, MM107, MM108, MM193, MM262, MM297, MM298, MM299, MM300, MM301, MM302, MM312, MM313, MM314, MM315, MM316, MM412, MM413, MM414, MM415, MM416, MM417, MM418, MM419, MM420, MM421, MM422, MM423, MM424, MM426, MM428; an anti-APLNR ligand antibody, preferably JN241, JN126, JN126Fc, JN241Fc, JN126FcP13, and an anti-APLNR antagonist antibody, preferably H2aM9232N.

**9.** The method of any one of the preceding claims, wherein the one or more agent(s) capable of inhibiting or blocking expression of an Apelin receptor or expression of one or more down-stream activators of Apelin signaling is/are (i) small interfering RNA molecule(s), or (ii) antisense oligonucleotide(s).

**11.** A composition comprising blood and immune cells or progenitor cells thereof, obtainable or obtained by the method of any one of the preceding claims, optionally further comprising a pharmaceutically acceptable carrier or a cell medium.

**12.** The composition of claim 11 for use in transplantation of immune and blood cells or progenitors thereof in a mammalian subject, preferably a human subject.

**13.** The composition for use according to claim 12 in treating a disease or disorder selected from the group consisting of hematologic disease, impaired hematopoiesis, pancytopenia, neutropenia, malignant disease autoimmune disorder, immunodeficiency, malignancy, and metabolic disease; wherein preferably the pancytopenia or neutropenia is caused by chemotherapy, myeloablation and/or radiation.

**14.** A kit for producing progenitor cells of blood and immune cells, comprising
one or more agents capable of inhibiting or blocking Apelin signaling, capable of inhibiting or blocking expression of an Apelin receptor and/or capable of inhibiting or blocking expression of one or more down-stream activators of Apelin signaling; and
instructions for use in a method of any one of the preceding claims and; optionally, a starting population of cells, said starting population comprises endothelial cells and/or progenitor cells thereof, growth factors and/or cell culture media.

**15.** A pharmaceutical composition comprising one or more agents capable of inhibiting or blocking Apelin signaling and a population of endothelial cells or progenitors of endothelial cells thereof, preferably the endothelial cells are arterial endothelial cells.
